# EUROPEAN PATENT APPLICATION

(11) **EP 4 672 128 A1**
(43) Date of publication of application: **31.12.2025**
(21) Application number: 24778765.8
(22) Date of filing: 13.02.2024
(51) Int. Cl.: G06Q 50/04, C12M 1/34, C12Q 1/02, G01N 21/65, G01N 33/15, G01N 33/483, G01N 33/68

(54) **QUALITY MONITORING DEVICE, METHOD FOR OPERATING QUALITY MONITORING DEVICE, AND PROGRAM FOR OPERATING QUALITY MONITORING DEVICE**

(30) Priority: 31.03.2023 JP 2023059234
(71) Applicant: FUJIFILM Corporation, Tokyo 106-8620 (JP)
(72) Inventor: SUGITA, Yui, Ashigarakami-gun, Kanagawa 258-8577 (JP); SASAKI, Yoshihiko, ashigara-shi, Kanagawa 250-0193 (JP); NAKAGAWA, Koyo, ashigara-shi, Kanagawa 250-0193 (JP)
(74) Representative: Hoffmann Eitle
(86) International application number: PCT/JP2024/004892
(87) International publication number: WO 2024/202620

(57) **Abstract**

A quality monitoring apparatus includes a processor configured to execute monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and model switching processing of switching the state prediction models during the monitoring processing based on step management information for managing at least one step included in the manufacturing process, in which the plurality of state prediction models have been trained before the monitoring processing is started, and the step management information includes at least one of step identification information, usage device information, or step quality information.

## Description

### BACKGROUND OF THE INVENTION

### 1. Field of the Invention

The technology of the present disclosure relates to a quality monitoring apparatus, an operation method of a quality monitoring apparatus, and an operation program of a quality monitoring apparatus.

### 2. Description of the Related Art

For example, a manufacturing process of a biopharmaceutical containing a biological molecule such as a protein, such as a monoclonal antibody, as an active ingredient is known. In such a manufacturing process, a suspension in which various components including the active ingredient are dispersed in liquid is often produced. It is important to monitor a state of a target component (for example, the protein or an impurity derived from the protein) in the suspension in a manufacturing line, in order to successfully lead the ongoing manufacturing process.

JP2022-512775A discloses a technology of predicting a concentration as a state of a target component in a manufacturing process. Specifically, in JP2022-512775A, a Raman spectrum of the suspension is measured in a manufacturing line, and a concentration of the target component is predicted from the Raman spectrum using a state prediction model.

### SUMMARY OF THE INVENTION

The prediction accuracy of the state prediction model is important for managing a quality of the manufacturing process. In the manufacturing process, a state of the liquid such as the suspension may fluctuate over time, and, in a case in which the fluctuation in the state is large, the prediction accuracy of the state prediction model may decrease. In the technology disclosed in JP2022-512775A, training data of the state prediction model is collected during the operation of the manufacturing process, and the state prediction model is updated with the collected training data, so that the decrease in prediction accuracy is suppressed.

However, in the technology disclosed in JP2022-512775A in which the training data is collected and the state prediction model is updated during the operation of the manufacturing process, for example, in the following cases, it may not be possible to cope with the decrease in prediction accuracy, and the quality management may be insufficient. That is, a case in which the manufacturing process includes a plurality of steps and the fluctuation in the state is large between the steps. In a case in which there is no margin in a time interval between the steps, there is a concern that the update of the state prediction model may not be completed in time in the method of JP2022-512775A. In such a case, the quality management may be insufficient.

One embodiment according to the technology of the present disclosure provides a quality monitoring apparatus, an operation method of a quality monitoring apparatus, and an operation program of a quality monitoring apparatus, which can appropriately manage quality of a manufacturing process of a biopharmaceutical as compared with the related art.

The present disclosure relates to a quality monitoring apparatus comprising: a processor configured to execute monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and model switching processing of switching the state prediction models during the monitoring processing based on step management information for managing at least one step included in the manufacturing process, in which the plurality of state prediction models have been trained before the monitoring processing is started, and the step management information includes at least one of step identification information for identifying at least one step included in the manufacturing process, usage device information of a usage device used in the step, or step quality information that is related to a quality of each step and that is acquirable in a case in which the step is performed at least once.

It is preferable that the model switching processing includes processing of switching the state prediction models based on the step identification information or the usage device before the one step included in the manufacturing process is started.

It is preferable that the model switching processing includes processing of switching the state prediction models based on quality information that is one of the step quality information and that is acquired during the step.

It is preferable that, in a case in which the manufacturing process includes a purification process of a target component contained in the liquid, the state of the liquid is a concentration of the target component contained in the liquid, and the quality information acquired during the step includes information that is related to the concentration of the target component in the liquid and that is acquired inline in the step.

It is preferable that the target component is an antibody.

It is preferable that the spectroscopic spectrum includes any one of an infrared spectrum, a fluorescence spectrum, or a Raman spectrum.

It is preferable that the processor is configured to output a prediction value of the state of the liquid at a first time interval set in advance, based on the spectroscopic spectrum.

It is preferable that the first time interval is 5 seconds or less.

It is preferable that the first time interval is 1 second or less.

It is preferable that the processor is configured to acquire the spectroscopic spectrum at a second time interval equal to or shorter than the first time interval, and output the prediction value based on a moving average value of a plurality of the acquired spectroscopic spectra.

It is preferable that the processor is configured to output a final quality of the step, indicating a final state of an entire liquid produced in the step, based on the prediction value.

It is preferable that the processor is configured to execute condition determination processing of determining process conditions of a next step based on the final quality of the step.

It is preferable that, in a case in which the manufacturing process includes a purification process of a target component contained in the liquid, the state of the liquid is a concentration of the target component contained in the liquid.

It is preferable that the target component is an antibody.

It is preferable that the state of the liquid is a state of a protein contained in the liquid.

The present disclosure relates to an operation method of a quality monitoring apparatus, the operation method comprising: executing monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and model switching processing of switching the state prediction models during the monitoring processing based on step management information for managing at least one step included in the manufacturing process, in which the plurality of state prediction models have been trained before the monitoring processing is started, and the step management information includes at least one of step identification information for identifying at least one step included in the manufacturing process, usage device information of a usage device used in the step, or step quality information that is related to the state of the liquid for each step and that is acquirable in a case in which the step is performed at least once.

The present disclosure relates to an operation program of a quality monitoring apparatus that causes a computer to function as the quality monitoring apparatus, the operation program causing the computer to execute: monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and model switching processing of switching the state prediction models during the monitoring processing based on step management information for managing at least one step included in the manufacturing process, in which the plurality of state prediction models have been created before the monitoring processing is started, and the step management information includes at least one of step identification information for identifying at least one step included in the manufacturing process, usage device information of a usage device used in the step, or step quality information that is related to the state of the liquid for each step and that is acquirable in a case in which the step is performed at least once.

According to the technology of the present disclosure, it is possible to appropriately monitor the quality of the manufacturing process of the biopharmaceutical as compared with the related art.

### BRIEF DESCRIPTION OF THE DRAWINGS

FIG. 1 is a diagram showing an overview of a manufacturing process of a biopharmaceutical.
FIG. 2 is a block diagram of a computer constituting a quality monitoring apparatus.
FIG. 3 is a block diagram of a processor constituting the quality monitoring apparatus.
FIG. 4 is a diagram showing process management information.
FIG. 5 is a diagram showing spectrum measurement data.
FIG. 6 is a diagram showing a neural network constituting a concentration prediction model.
FIG. 7 is a diagram showing the concentration prediction model and training data.
FIG. 8 is a diagram showing a state in which prediction is performed at a regular period.
FIG. 9 is a diagram showing a monitoring screen showing a predicted antibody concentration in time series.
FIG. 10 is a flowchart showing a monitoring processing procedure.
FIG. 11 is a diagram showing a method of acquiring a final antibody concentration.
FIG. 12 is a diagram showing a plurality of items predicted by the concentration prediction model.
FIG. 13 is a diagram showing another example of the monitoring screen.
FIG. 14 is a diagram showing process management information of a modification example of a first embodiment.
FIG. 15 is a diagram showing a concentration prediction model and training data of the modification example of the first embodiment.
FIG. 16 is a diagram showing a processing procedure of the modification example of the first embodiment.
FIG. 17 is a diagram showing process management information of a second embodiment.
FIG. 18 is a diagram showing a processing procedure of the second embodiment.
FIG. 19 is a diagram showing a temporal change in absorbance and a model switching timing.
FIG. 20 is a diagram showing process management information of a modification example of the second embodiment.
FIG. 21 is a diagram showing a processing procedure of the modification example of the second embodiment.
FIG. 22 is a diagram showing a temporal change in antibody concentration and a model switching timing.
FIG. 23 is a diagram showing a method of obtaining the final antibody concentration from a prediction value.
FIG. 24 is a diagram showing a moving average of the spectrum measurement data.

### DESCRIPTION OF THE PREFERRED EMBODIMENTS

### [First Embodiment]

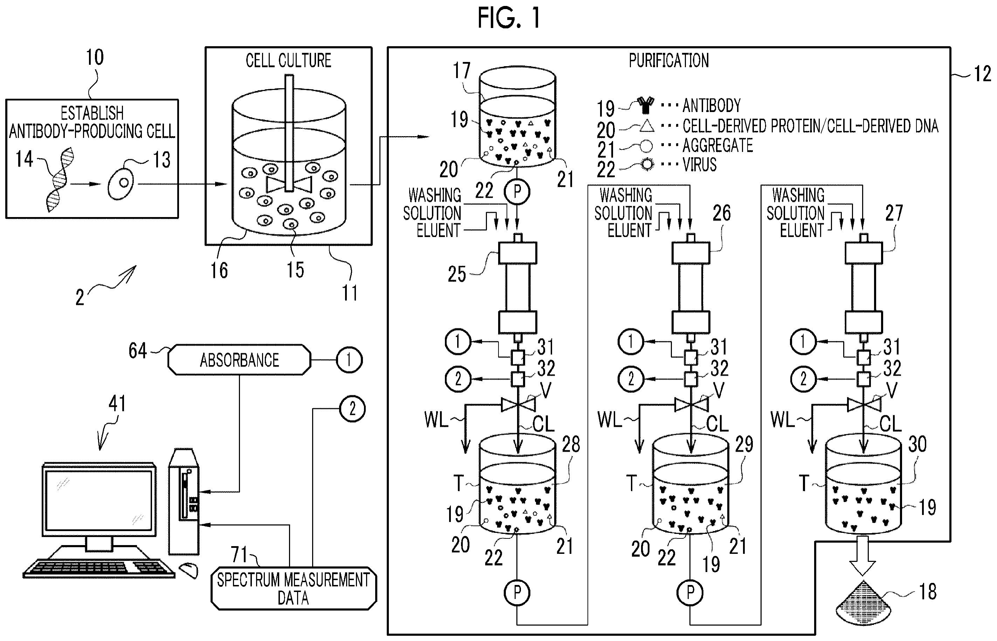

As shown in FIG. 1 as an example, a manufacturing process 2 of a biopharmaceutical, to which a quality monitoring apparatus 41 according to the technology of the present disclosure is applied, is roughly divided into a first process 10, a second process 11, and a third process 12. The first process 10 is a process of incorporating an antibody gene 14 into a host cell 13 such as Chinese hamster ovary (CHO) cells to establish an antibody-producing cell 15. The second process is a process of culturing the antibody-producing cell 15 in a culture tank 16.

The third process 12 is a purification process of purifying a drug substance 18 of the biopharmaceutical from a culture supernatant liquid 17. The culture supernatant liquid 17 is a solution obtained by removing cells from a culture liquid in the culture tank 16 for which the second process 11 ends. A protein, that is, an antibody 19, which is produced by the antibody-producing cell 15, is dispersed in the culture supernatant liquid 17. The antibody 19 is, for example, a monoclonal antibody, and is an active ingredient of the biopharmaceutical. In addition, in addition to the antibody 19, impurities such as a cell-derived protein/cell-derived deoxyribonucleic acid (DNA) 20 and an aggregate 21 of the antibody 19, or a virus 22 are also dispersed in the culture supernatant liquid 17.

An immunoaffinity chromatography device 25, a cation exchange chromatography device 26, and an anion exchange chromatography device 27 are used in the third process 12.

The culture supernatant liquid 17 is introduced into the immunoaffinity chromatography device 25. For example, a pump P is provided upstream of the immunoaffinity chromatography device 25. The culture supernatant liquid 17 is introduced into the immunoaffinity chromatography device 25 by driving the pump P. The immunoaffinity chromatography device 25 extracts the antibody 19 from the culture supernatant liquid 17 by using a column in which a ligand such as a protein A having an affinity for the antibody 19 is immobilized on a carrier, and thereby generating a first purified liquid 28. Here, a purification step by the immunoaffinity chromatography device 25 will be referred to as an immunoaffinity chromatography step. A recovery container T is provided downstream of the immunoaffinity chromatography device 25, and the first purified liquid 28 is recovered in the recovery container T.

A valve V is provided on a pipe line between the immunoaffinity chromatography device 25 and the recovery container T. The valve V switches a flow passage of the liquid flowing out from the immunoaffinity chromatography device 25 between a recovery line CL toward the recovery container T and a waste liquid line WL.

The immunoaffinity chromatography step includes an adsorption step, a washing step, and an elution step as sub-steps. The adsorption step is a step of specifically adsorbing the antibody 19 to the column by introducing the culture supernatant liquid 17 into the column. The washing step is a step of washing impurities other than the antibody 19, which are non-specifically adsorbed to the column, by introducing a washing solution into the column. The elution step is a step of introducing an acidic eluent into the column to peel off the antibody 19 that is specifically adsorbed to the column and elute the antibody 19 into the eluent. In the adsorption step and the washing step, the flow passage is switched by the valve V such that the liquid from the immunoaffinity chromatography device 25 flows to the waste liquid line WL. In the adsorption step and the washing step, most of the components contained in the liquid flowing out from the immunoaffinity chromatography device 25 are impurities. This liquid is discharged as waste liquid through the waste liquid line WL.

In the elution step, the flow passage is switched by the valve V such that the liquid from the immunoaffinity chromatography device 25 flows to the recovery line CL. The first purified liquid 28 containing the antibody 19 extracted through the column flows out from the immunoaffinity chromatography device 25 in the elution step, and is recovered in the recovery container T through the recovery line CL.

Although not shown, the first purified liquid 28 is subjected to a treatment for inactivating the virus 22 (hereinafter, referred to as virus inactivation treatment).

In addition, an ultraviolet detector 31 (hereinafter, referred to as UV detector) and a Raman spectrometer 32 are provided on a pipe line between the immunoaffinity chromatography device 25 and the valve V. The UV detector 31 and the Raman spectrometer 32 are sensors that measure a measurement value related to a state of the liquid flowing through the pipe line, and are sensors that sense, inline, a characteristic value related to a quality of the liquid in the pipe line while the third process 12 is in operation.

The Raman spectrometer 32 includes a flow cell, a probe, and an analyzer. The flow cell of the Raman spectrometer 32 is connected to a pipe line on a downstream side of the UV detector 31. The liquid, which has passed through the UV detector 31, flows through the flow cell. The probe is connected to the flow cell. A Raman spectrometer 32 is a device that evaluates the substance by using characteristics of Raman scattered light. In a case in which the substance is irradiated with excitation light, Raman scattered light having a different wavelength from the excitation light is generated by an interaction between the excitation light and the substance. A difference in wavelength between the excitation light and the Raman scattered light corresponds to an energy of molecular vibration possessed by the substance. Therefore, the Raman scattered light having different wave numbers can be obtained between the substances having different molecular structures. It is preferable to use, out of a Stokes ray and an anti-Stokes ray, the Stokes ray as the Raman scattered light. The spectrum of the Raman scattered light, that is, the Raman spectrum is an example of a "spectroscopic spectrum" according to the technology of the present disclosure.

In the Raman spectrometer 32, the probe emits the excitation light from an emission port at a distal end thereof to the liquid flowing through the flow cell. Then, the Raman scattered light generated by the interaction between the excitation light and the substance in the liquid is received by a light-receiving portion disposed at the distal end. The probe outputs the received Raman scattered light to the analyzer. In the present example, laser light is used as the excitation light, the output of the laser light is 500 mW, an excitation wavelength is 785 nm, and an irradiation time for one time is 1 second. The Raman spectrometer 32 acquires the Raman spectrum at an interval of 1 second. The interval of 1 second is an example of a "second time interval" according to the technology of the present disclosure. The second time interval is a time interval equal to or shorter than the first time interval described later. Although not particularly limited, the time is preferably 5 seconds or less and more preferably 1 second or less in order to monitor the short-time liquid concentration fluctuation.

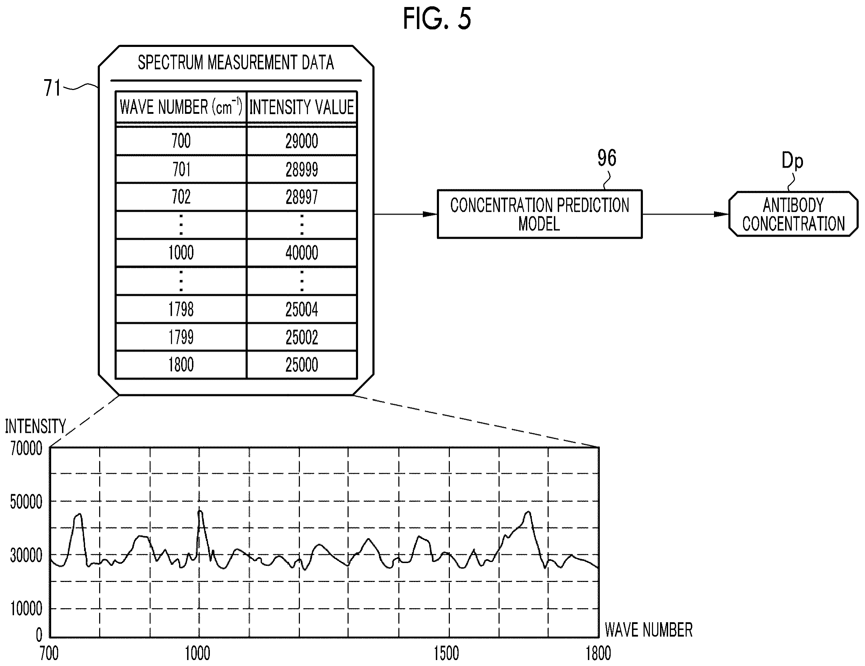

In the Raman spectrometer 32, the analyzer decomposes the Raman spectrum for each wave number and derives an intensity value of the Raman spectrum for each wave number to generate a spectrum measurement data 71 (see FIG. 5).

As shown in FIG. 5 as an example, the spectrum measurement data 71 is data in which the intensity value of the Raman scattered light is registered for each wave number. In FIG. 5, the spectrum measurement data 71 is data in which the intensity values of the scattered light in a range of wave numbers of 700 cm⁻¹ to 1800 cm⁻¹ are derived in increments of 1 cm⁻¹. A graph shown in the lower part of FIG. 5 is obtained by plotting the intensity value of the spectrum measurement data 71 for each wave number and connecting the plots with lines, and visualizes the Raman spectrum.

The UV detector 31 irradiates the liquid from the column with detection light, and measures an absorbance (light absorption amount) 64 of the substance in the liquid. The detection light is ultraviolet light and/or visible light (light having a wavelength of 190 nm to 800 nm) having a wavelength corresponding to the antibody 19 and the aggregate 21, and, in the present example, the detection light is ultraviolet light having a wavelength of 280 nm. An acquisition interval of the UV detector 31 is also set to 1 second, which is the same as the Raman spectrum, as an example. The absorbance 64 serves as a guideline for the antibody concentration, and is effective as a quality monitoring item. However, since the detection light has sensitivity to the cell-derived protein and the cell-derived DNA 20 in addition to the aggregate 21 other than the antibody 19, an accurate antibody concentration cannot be measured. Therefore, the prediction of the antibody concentration based on the Raman spectrum is performed as will be described later.

The first purified liquid 28 after the virus inactivation treatment is introduced into the cation exchange chromatography device 26. The cation exchange chromatography device 26 extracts the antibody 19 from the first purified liquid 28 by using a column of which a stationary phase is a cation exchanger, to generate a second purified liquid 29. Here, a purification step by the cation exchange chromatography device 26 will be referred to as a cation exchange chromatography step. As in the immunoaffinity chromatography step, the cation exchange chromatography step includes an adsorption step, a washing step, and an elution step as sub-steps. In addition, in the cation exchange chromatography step, the configurations and functions of the valve V, the recovery line CL, the waste liquid line WL, the UV detector 31, and the Raman spectrometer 32 are the same as those in the immunoaffinity chromatography step.

The second purified liquid 29 is introduced into the anion exchange chromatography device 27. The anion exchange chromatography device 27 extracts the antibody 19 from the second purified liquid 29 by using a column of which a stationary phase is an anion exchanger, to generate a third purified liquid 30. Here, a purification step by the anion exchange chromatography device 27 will be referred to as an anion exchange chromatography step. Although not shown, a treatment of removing the virus is performed on the third purified liquid 30. Thereafter, the third purified liquid 30 is subjected to a concentration/filtration treatment by an ultrafiltration (UF) and a diafiltration (DF), so that the drug substance 18 is purified. As in the immunoaffinity chromatography step, the anion exchange chromatography step includes an adsorption step, a washing step, and an elution step as sub-steps. In addition, in the anion exchange chromatography step, the configurations and functions of the valve V, the recovery line CL, the waste liquid line WL, the UV detector 31, and the Raman spectrometer 32 are the same as those in the immunoaffinity chromatography step.

By sequentially performing a component separation treatment using such a plurality of types of the chromatography devices 25 to 27, impurities and the virus 22 are gradually removed, and a purity of the antibody 19 is gradually increased. The antibody 19 is an example of a "target component" and a "protein" according to the technology of the present disclosure. The target component refers to a component to be purified in each step by the plurality of types of chromatography devices 25 to 27.

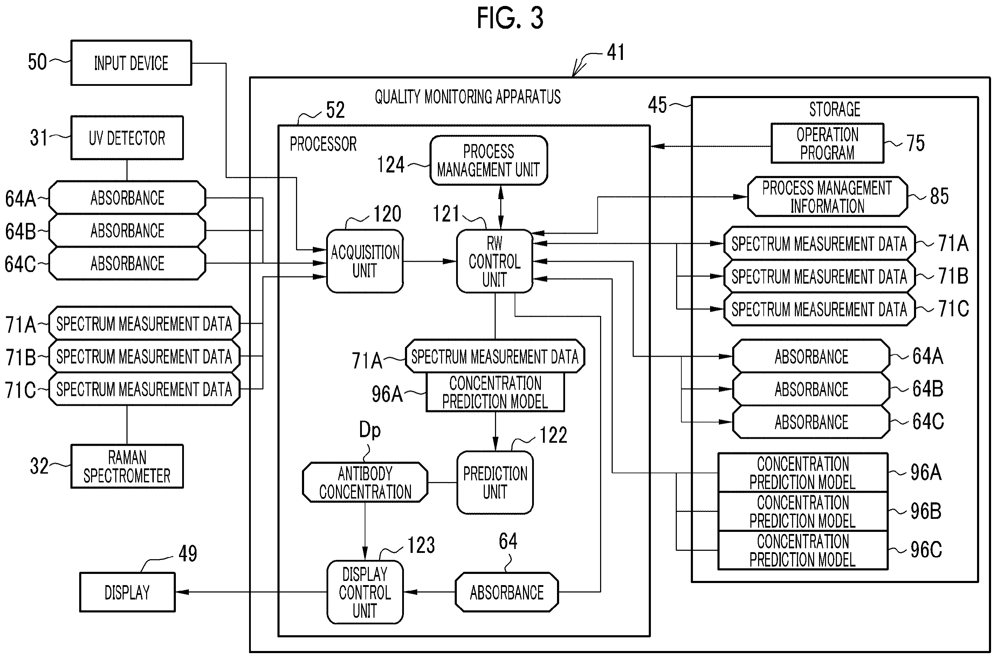

The quality monitoring apparatus 41 is an example of a "quality monitoring apparatus" according to the technology of the present disclosure. The quality monitoring apparatus 41 has a function of monitoring a quality of the third process 12 in the manufacturing process 2. The quality of the third process 12 is ultimately the quality of the drug substance 18, but the quality of the third process 12 includes the quality of the liquid flowing out from each of the chromatography devices 25 to 27, such as the first purified liquid 28, the second purified liquid 29, and the third purified liquid 30, which are obtained in the process of reaching the drug substance 18. The quality monitoring apparatus 41 executes monitoring processing of monitoring the quality of the third process 12 by using a state prediction model that predicts the state of the liquid flowing out from each of the chromatography devices 25 to 27. The "state of the liquid" is an indicator indicating the physicochemical characteristics of the liquid, and, in the present example, is an antibody concentration Dp (see FIG. 3 and the like), which is the concentration of the antibody 19. The antibody concentration Dp is an example of a concentration of the target component contained in the liquid according to the technology of the present disclosure.

The quality monitoring apparatus 41 uses a concentration prediction model 96 (see FIGS. 3 and 5 and the like) as the state prediction model. The concentration prediction model 96 predicts the antibody concentration Dp, which is an example of the state of the liquid related to the quality of the third process 12, using the spectrum measurement data 71 representing the spectroscopic spectrum acquired inline as input data. The concentration prediction model 96 is an example of a "state prediction model" according to the technology of the present disclosure.

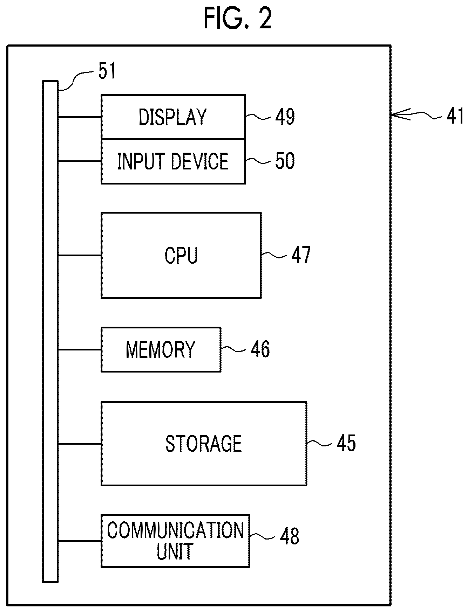

As shown in FIG. 2 as an example, the quality monitoring apparatus 41 is configured by a computer such as a personal computer and a server computer. The computer constituting the quality monitoring apparatus 41 comprises a storage 45, a memory 46, a central processing unit (CPU) 47, a communication unit 48, a display 49, and an input device 50. These units are connected to each other via a busline 51.

The storage 45 is a hard disk drive built in the computer or connected to the computer through a cable or a network. Alternatively, the storage 45 is a disk array in which a plurality of hard disk drives are connected together. The storage 45 stores a control program such as an operating system, various application programs, and various data associated with these programs. A solid state drive may be used instead of the hard disk drive.

The memory 46 is a work memory for the CPU 47 to execute processing. The CPU 47 loads the program stored in the storage 45 into the memory 46 to execute the processing in accordance with the program. As a result, the CPU 47 integrally controls the units of the computer. The CPU 47 is an example of a "processor" according to the technology of the present disclosure. The memory 46 may be built in the CPU 47.

The communication unit 48 is a network interface that performs control of transmitting various types of information via a network 42 and the like. The display 49 displays various screens. The various screens have an operation function by a graphical user interface (GUI). The computer constituting the quality monitoring apparatus 41 receives an input of an operation instruction from the input device 50 via the various screens. The input device 50 is, for example, a keyboard, a mouse, a touch panel, and a microphone for voice input.

As shown in FIG. 3 as an example, an operation program 75 is stored in the storage 45 of the quality monitoring apparatus 41. The operation program 75 is an application program causing the computer to function as the quality monitoring apparatus 41. That is, the operation program 75 is an example of an "operation program of a quality monitoring apparatus" according to the technology of the present disclosure. The storage 45 stores process management information 85 and a plurality of concentration prediction models 96A, 96B, and 96C as the concentration prediction model 96, in addition to the operation program 75.

The plurality of concentration prediction models 96A, 96B, and 96C are used differently for each step. The concentration prediction model 96A is used to predict the antibody concentration Dp in the immunoaffinity chromatography step. The concentration prediction model 96B is used to predict the antibody concentration Dp in the cation exchange chromatography step. The concentration prediction model 96C is used to predict the antibody concentration Dp in the cation exchange chromatography step. Hereinafter, in a case in which it is not necessary to distinguish between three types of data, the three types of data are simply referred to as the concentration prediction model 96.

In a case in which the operation program 75 is activated, the CPU 47 of the computer constituting the quality monitoring apparatus 41 functions as a processor 52 including an acquisition unit 120, an RW control unit 121, a prediction unit 122, a display control unit 123, and a process management unit 124 in cooperation with the memory 46 and the like.

The acquisition unit 120 acquires the spectrum measurement data 71 from the Raman spectrometer 32. The acquisition unit 120 outputs the spectrum measurement data 71 to the RW control unit 121. In FIG. 3, as the spectrum measurement data 71, spectrum measurement data 71A, 71B, and 71C are described by distinguishing the data acquired in each of the chromatography steps. The spectrum measurement data 71A is data acquired in the immunoaffinity chromatography step, the spectrum measurement data 71B is data acquired in the cation exchange chromatography step, and the spectrum measurement data 71C is data acquired in the anion exchange chromatography device 27. Hereinafter, in a case in which it is not necessary to distinguish between the three types of data, the three types of data are simply referred to as the spectrum measurement data 71.

Further, the acquisition unit 120 acquires the absorbance 64 from the UV detector 31. The acquisition unit 120 outputs the absorbance 64 to the RW control unit 121. As the absorbance 64, absorbances 64A, 64B, and 64C are described by distinguishing the data acquired in each of the chromatography steps. The absorbance 64A is data acquired in the immunoaffinity chromatography step, the absorbance 64B is data acquired in the cation exchange chromatography step, and the absorbance 64C is data acquired in the anion immunoaffinity chromatography step. Hereinafter, in a case in which it is not necessary to distinguish between the three types of data, the three types of data are simply referred to as the absorbance 64.

Furthermore, the acquisition unit 120 acquires various operation instructions from the input device 50. The various operation instructions include an instruction to start each step of the third process 12.

The RW control unit 121 controls the storage of various types of data into the storage 45 and the readout of various types of data stored in the storage 45. The RW control unit 121 stores the spectrum measurement data 71 and the absorbance 64 from the acquisition unit 120 in the storage 45. The spectrum measurement data 71 and the absorbance 64 are stored with an acquisition time added as a timestamp.

In addition, the RW control unit 121 reads out the process management information 85 from the storage 45 and outputs the readout process management information 85 to the process management unit 124. The RW control unit 121 reads out the process management information 85, the concentration prediction model 96, and the spectrum measurement data 71 from the storage 45, and outputs the readout concentration prediction model 96 and the readout spectrum measurement data 71 to the prediction unit 122. The concentration prediction models 96A to 96C and the spectrum measurement data 71A to 71C are selectively output to the prediction unit 122 in accordance with the step. FIG. 3 shows an example in which the concentration prediction model 96A and the spectrum measurement data 71A used in the immunoaffinity chromatography step are output. Further, the RW control unit 121 outputs the absorbance 64 to the display control unit 123.

The prediction unit 122 applies the spectrum measurement data 71 to the concentration prediction model 96 to output the antibody concentration Dp from the concentration prediction model 96. The prediction unit 122 outputs the predicted antibody concentration Dp to the display control unit 123.

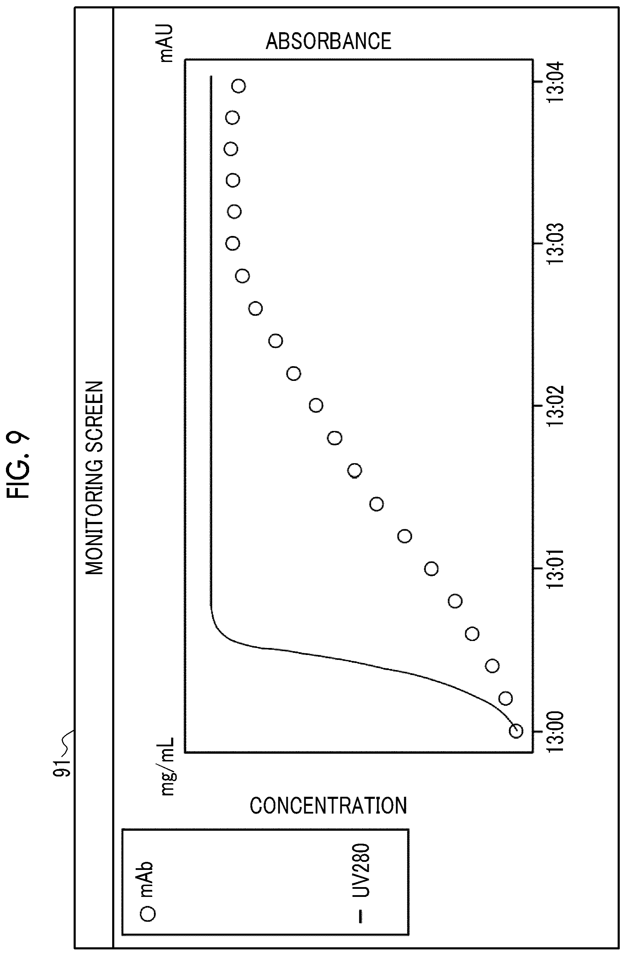

The display control unit 123 controls the display of the various screens on the display 49. For example, the display control unit 123 performs control of displaying a monitoring screen 91 (see FIG. 9 and the like) on the display 49. In the monitoring screen 91, the antibody concentration Dp and the absorbance 64 acquired inline during the execution of each step of the third process 12 are displayed in real time.

In a case in which the third process 12 is executed, the process management unit 124 executes the monitoring processing and model switching processing by controlling the driving of the usage device such as the pump P and the valve V and integrally controlling the units of the processor 52. The monitoring processing is processing of acquiring the spectrum measurement data 71 and the absorbance 64, predicting the antibody concentration Dp using the concentration prediction model 96, and displaying the predicted antibody concentration Dp and the absorbance 64 on the display 49. As a result, a user, such as an operator, can check the quality of the third process 12.

The process management unit 124 executes the model switching processing of switching the concentration prediction models 96 during the monitoring processing, based on the process management information 85.

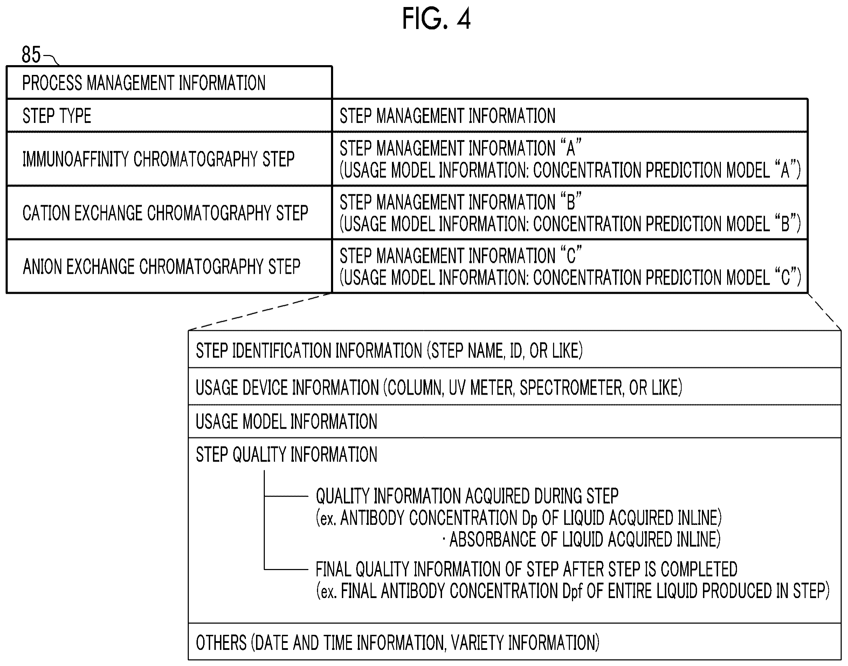

As shown in FIG. 4 as an example, the process management information 85 includes the step management information of each chromatography step included in the third process 12, that is, the step management information of the immunoaffinity chromatography step (marked with "A" in FIG. 4), the step management information of the cation exchange chromatography step (marked with "B" in FIG. 4), and the step management information of the anion exchange chromatography step (marked with "C" in FIG. 4).

Each step management information includes step identification information, usage device information, step quality information, usage model information, and other information. The step identification information is information for identifying each chromatography step included in the third process 12, and specifically, is a step name, identification information (ID) of the step, and the like. The usage device information is information on the usage device (the columns of the chromatography devices 25 to 27, the UV detector 31, the Raman spectrometer 32, the pump P, the valve V, and the like) used in each step of the third process 12. The step quality information is information that is related to the quality of each step and that can be acquired in a case in which each chromatography step is performed at least once. Specifically, there are quality information acquired during the step, final quality information of the step after the completion of the step, and the like. The quality information acquired during the step includes the antibody concentration Dp that is acquired inline, the absorbance 64 that is acquired inline, and the like.

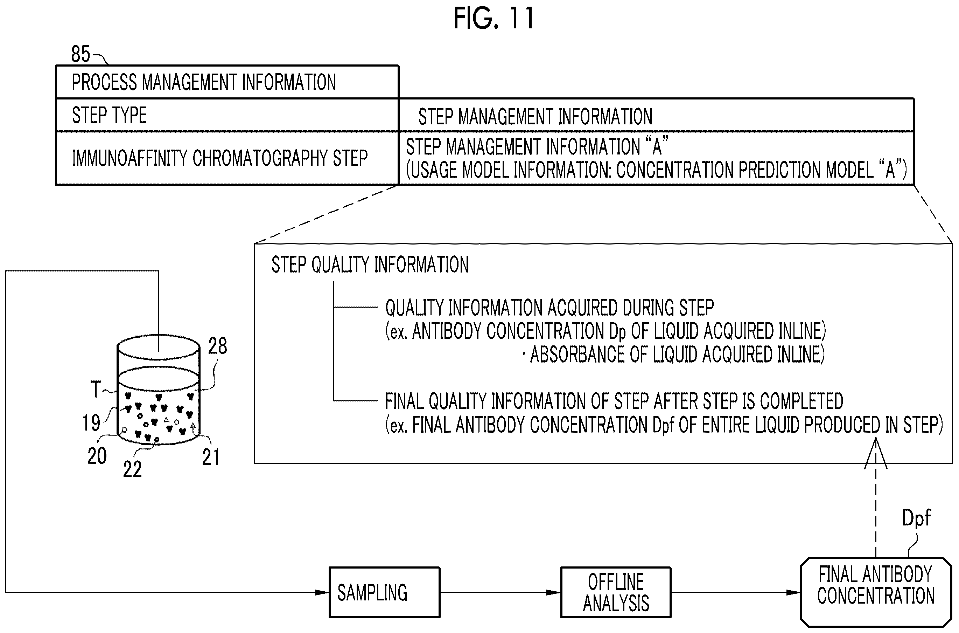

The final quality information is, for example, a final antibody concentration Dpf (see FIG. 11 and the like) of the entire liquid produced in the step, which is the final quality. More specifically, in a case of the immunoaffinity chromatography step, the average antibody concentration refers to an average antibody concentration of a total amount of the first purified liquid 28 recovered in the recovery container T after the completion of the step. The antibody concentration Dp acquired inline is, so to speak, an instantaneous value and fluctuates depending on the acquisition timing. On the other hand, the final antibody concentration Dpf, which is the final quality, is a final value that does not fluctuate and is an average value of the fluctuating antibody concentration Dp. In a case in which the first purified liquid 28 is sent to the next step without being recovered in the recovery container T, the final antibody concentration Dpf is an average antibody concentration Dp of the total amount of the first purified liquid 28 sent to the next step.

The usage model information is information on the concentration prediction model 96 used in each step. In the present example, the model used in the immunoaffinity chromatography step is the concentration prediction model 96A (a name in the step management information is a concentration prediction model "A"). The model used in the cation exchange chromatography step is the concentration prediction model 96B (a name in the step management information is a concentration prediction model "B"). The model used in the anion exchange chromatography step is the concentration prediction model 96C (a name in the step management information is a concentration prediction model "C").

The other information includes date and time information, variety information, and the like. The variety information is, for example, information on the variety of the antibody 19 as the target component.

As described above, the process management information 85 of the present example is information in which the information registered by the operator, such as the step identification information, the usage device information, and the usage model information, and the information acquired in a case in which the step is performed once, such as the step quality information, are mixed.

The step management information, the step identification information, the usage device information, and the step quality information shown in FIG. 4 are examples of "step management information", "step identification information", "usage device information", and "step quality information" according to the technology of the present disclosure. In the present example, although the example has been described in which a plurality of step management information are collected as one of the process management information 85, each step management information may be divided for each step.

In the present example, the process management unit 124 executes the model switching processing based on the step identification information before one step included in the third process 12 is started. Specifically, the process management unit 124 sets the concentration prediction model 96A with reference to the step identification information before the immunoaffinity chromatography step is started. Then, after the immunoaffinity chromatography step is completed and before the cation exchange chromatography step is started, the concentration prediction model 96B is set with reference to the step identification information. Further, after the cation exchange chromatography step is completed and before the anion exchange chromatography step is started, the concentration prediction model 96C is set with reference to the step identification information.

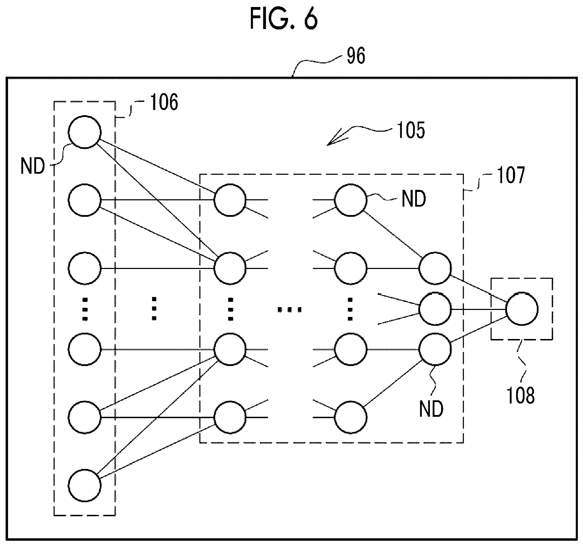

As an example, as shown in FIGS. 5 and 6, the concentration prediction model 96 is constructed by a neural network 105. As described above, the concentration prediction model 96 is a machine learning model. The neural network 105 includes, as is well known, an input layer 106, an intermediate layer (also referred to as a hidden layer) 107, and an output layer 108. The input layer 106, the intermediate layer 107, and the output layer 108 each include a plurality of nodes ND. A coefficient indicating the connection strength of the respective nodes ND is set between the node ND of the input layer 106 and the node ND of the intermediate layer 107, between the nodes ND of the intermediate layer 107, and between the node ND of the intermediate layer 107 and the node ND of the output layer 108. A suitable activation function such as a linear function or a rectified linear unit (ReLU) function is set for the node ND of the output layer 108.

The intensity value of each wave number of the spectrum measurement data 71 shown in FIG. 5 is input as the input data to each node ND of the input layer 106. In addition, the antibody concentration Dp, which is a concentration prediction value of the antibody 19, is output from the node ND of the output layer 108.

In the concentration prediction model 96, in a case in which a fluctuation range of the predicted antibody concentration Dp is large, the prediction accuracy may decrease. Since the third process 12 is a process of gradually increasing the antibody concentration Dp for each step of the chromatography steps, the fluctuation range of the antibody concentration Dp is large between the steps. Therefore, in the quality monitoring apparatus 10, the decrease in the prediction accuracy is suppressed by switching the concentration prediction models 96 for each step.

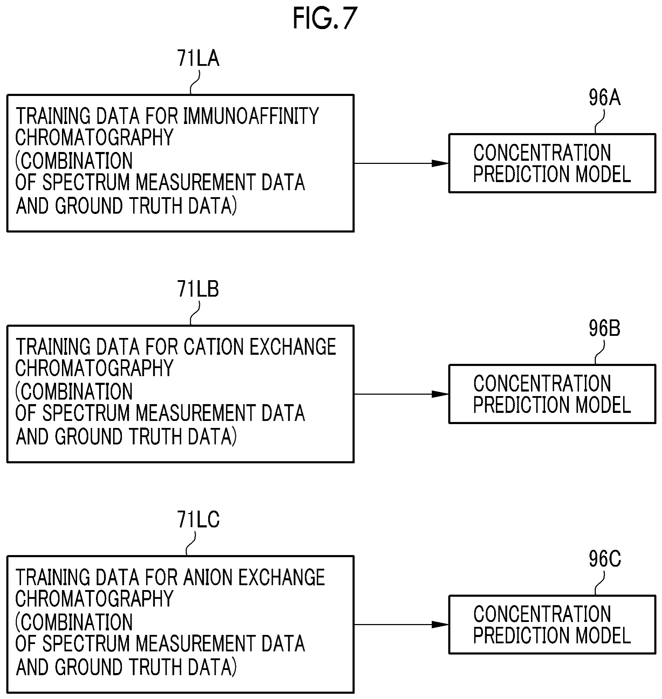

Further, as shown in FIG. 7 as an example, the plurality of concentration prediction models 96A to 96C used in each chromatography step have been trained before the monitoring processing of the third process 12 is started. Each of the concentration prediction models 96A to 96C has been trained using different training data 71LA to 71LC in accordance with a range of the antibody concentration Dp predicted by each of the concentration prediction models 96A to 96C. The training data 71LA of the concentration prediction model 96A is training data for the immunoaffinity chromatography step, and is training data in accordance with the range of the antibody concentration Dp in the immunoaffinity chromatography step. The training data 71LA is composed of the spectrum measurement data 71 acquired for training and ground truth data of the antibody concentration Dp corresponding to the spectrum measurement data 71. As a method of creating the training data 71LA, for example, a method disclosed in WO2022/209422A may be used. That is, a plurality of first purified liquids 28 having different antibody concentrations Dp are created, and the spectrum measurement data 71 of the created plurality of first purified liquids 28 is acquired by the Raman spectrometer 32. The antibody concentration Dp, which is ground truth data of the plurality of first purified liquids 28, is acquired using, for example, a method such as high performance liquid chromatography (HPLC). A plurality of training data 71LA are created by a plurality of combinations of the spectrum measurement data 71 and the antibody concentration Dp that is the ground truth data. In order to improve the generalization performance of the concentration prediction model 96A for an unknown liquid, it is preferable that, as a plurality of first purified liquids 28, the first purified liquids 28 are prepared in which the concentration of impurities or the like is changed in various ways in addition to the antibody concentration Dp, and the corresponding training data 71LA is created.

In addition, in a case in which the plurality of training data 71LA are created, it is preferable to use a part of the plurality of training data 71LA as training data and to use the other part of the plurality of training data 71LA as test data for verifying the prediction accuracy. For the verification using the test data, for example, an evaluation value such as a root-mean-square error (RMSE) between the prediction value of the antibody concentration Dp and the ground truth data is used. Furthermore, the evaluation value of the verification result using the test data may be recorded in the storage 45 in association with each of the trained concentration prediction models 96A to 96C. For example, a plurality of concentration prediction models 96A used in the same step may be prepared. In such a case, for example, the evaluation value of the prediction accuracy may be used as an indicator for selecting the concentration prediction model 96A, such as using the concentration prediction model 96A with the highest evaluation value.

The training data 71LB of the concentration prediction model 96B is training data for the cation exchange chromatography step, and is training data in accordance with the range of the antibody concentration Dp in the cation exchange chromatography step. The training data LC of the concentration prediction model 96C is training data for the anion exchange chromatography step, and is training data in accordance with the range of the antibody concentration Dp in the anion exchange chromatography step. A method of creating the training data 71LB and the training data 71LC is also the same as the method of creating the training data 71LA.

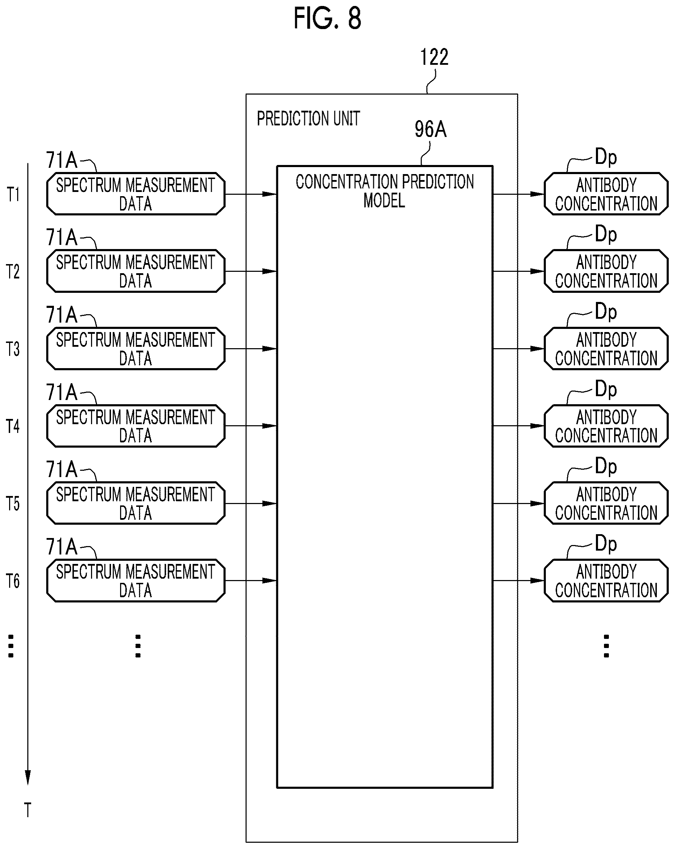

As an example, as shown in FIG. 8, in the immunoaffinity chromatography step, the prediction unit 122 outputs the antibody concentration Dp at a regular period using the concentration prediction model 96A. T1, T2, T3, T4, T5, T6, and the like are prediction times at which the antibody concentration Dp is predicted, and a time interval at each prediction time is a first time interval. The first time interval is an example of a "first time interval" according to the technology of the present disclosure. The first time interval is, specifically, 1 second. The first time interval is not particularly limited, but in order to monitor the short-term concentration fluctuation, the first time interval is preferably 5 seconds or less and more preferably 1 second or less, as described above. The first time interval, which is the prediction interval of the antibody concentration Dp, is rate-limited by the second time interval, which is the acquisition interval of the spectrum measurement data 71A of the Raman spectrometer 32. Therefore, the second time interval is set to a time equal to or shorter than the first time interval, as described above. In the example of FIG. 8, the first time interval and the second time interval are the same, that is, 1 second.

The antibody concentration Dp output by the prediction unit 122 at the first time interval is stored in the storage 45 with a timestamp that is a time of prediction. The spectrum measurement data 71A and the absorbance 64A are stored in the storage 45 with the timestamp. As a result, the spectrum measurement data 71A, the absorbance 64A, and the antibody concentration Dp are each stored in the storage 45 as time-series data.

It is preferable that the second time interval, which is the acquisition interval of the spectrum measurement data 71, and the first time interval, which is the prediction interval of the antibody concentration Dp, are the same as each other in consideration of the ease of comparison between the two data, but the second time interval and the first time interval may be different from each other. In such a case, it is preferable to perform adjustment such as thinning-out processing in accordance with the time-series data having a shorter time interval. In addition, in a case in which there is the timestamp, it is also possible to arrange two data in time series in a form in which the two data can be collated with each other based on the timestamp.

In the cation exchange chromatography step as well, as in the immunoaffinity chromatography step, the prediction unit 122 outputs the antibody concentration Dp at a preset first time interval using the concentration prediction model 96B. Then, the spectrum measurement data 71B, the absorbance 64B, and the antibody concentration Dp are stored in the storage 45 as time-series data. In the anion exchange chromatography step as well, the same prediction is performed, and the spectrum measurement data 71C, the absorbance 64C, and the antibody concentration Dp are stored in the storage 45 as time-series data.

As shown in FIG. 9 as an example, the display control unit 123 outputs the monitoring screen 91 on which a graph showing the temporal change in each of the absorbance 64 and the antibody concentration Dp is displayed based on the time-series data of the absorbance 64 and the antibody concentration Dp. The graph showing the temporal change is updated each time the latest antibody concentration Dp is acquired. The monitoring screen 91 displays, for example, the temporal change in the antibody concentration Dp in the elution step in the immunoaffinity chromatography step. In FIG. 9, the antibody concentration Dp is indicated by monoclonal Antibody (mAb). In addition, in FIG. 9, UV 280 indicates the absorbance 64 of the UV detector 31 in which ultraviolet rays having a wavelength of 280 nm are used as the detection light. In the elution step, since the antibody 19 adsorbed to the column of the immunoaffinity chromatography device 25 is eluted to flow out into the pipe line, the antibody concentration DP starts to increase. The antibody 19 adsorbed to the column is eventually reduced, and thus the antibody 19 also starts to be reduced.

As shown in a solid line graph, the absorbance 64 indicates a concentration higher than the antibody concentration Dp. This is because the detection light of the UV detector 31 is also sensitive to impurities other than the antibody 19 and the like. The example of FIG. 9 shows a state in which the absorbance 64 is saturated shortly after the antibody 19 starts to increase.

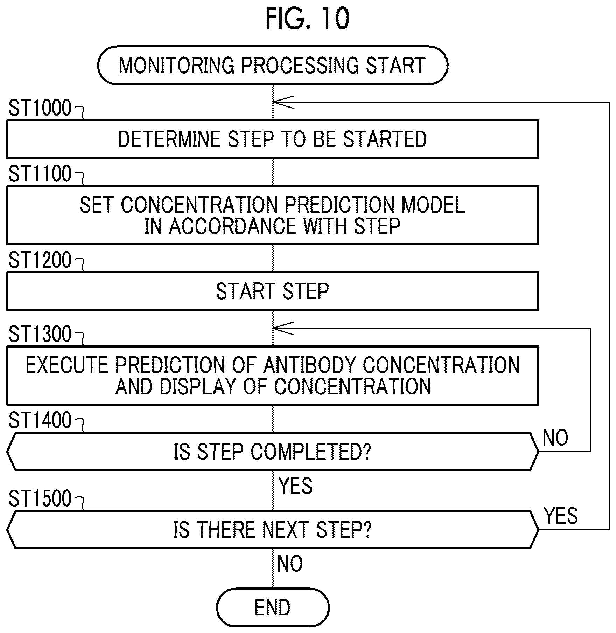

Next, an operation of the configuration described above will be described with reference to a flowchart shown in FIG. 10 as an example. The flowchart shown in FIG. 10 shows a procedure of the monitoring processing executed by the quality monitoring apparatus 41.

In the storage 45 of the quality monitoring apparatus 41, the trained concentration prediction models 96A to 96C are stored before the third process 12 is started. In this state, the third process 12 is started, and, at the same time, the monitoring processing is started. In a case in which the monitoring processing is started, in step ST1000, the processor 52 determines the step to be started. The step determination is performed, for example, based on an operation instruction to designate the name of the step to be started by the operator. For example, in a case in which the immunoaffinity chromatography step is started, the step name of the immunoaffinity chromatography step is designated as the step name.

In step ST1100, the processor 52 sets the concentration prediction model 96A corresponding to the designated step with reference to the step management information. In step ST1200, the processor 52 starts the driving of the pump P or the like based on the operation instruction to start the step, and starts the step.

In step ST1300, the processor 52 acquires the absorbance 64 and the spectrum measurement data 71, and predicts the antibody concentration Dp based on the spectrum measurement data 71. In addition, the processor 52 displays the temporal change in the absorbance 64 and the antibody concentration Dp on the display 49 through the monitoring screen 91.

In step ST1400, the processor 52 determines whether the step is completed. The determination of whether the step is completed is performed, for example, based on whether the antibody concentration Dp is equal to or less than a preset threshold value after the elution step is started. A case in which the antibody concentration Dp is equal to or less than the threshold value means that the elution of the antibody 19 from the column is close to the end. In a case in which the elution step is continued in a state in which the antibody concentration Dp is decreased, the final antibody concentration Dpf of the first purified liquid 28 also decreases. Therefore, the processor 52 determines whether the step is completed, based on the threshold value. In a case in which the step is not completed (NO in step ST1400), the processing of step ST1300 is continued. In a case in which the step is completed (YES in step ST1400), the processor 52 proceeds to step ST1500 and determines whether there is a next step.

In step ST1500, in a case in which there is a next step (YES in step ST1500), the processor 52 returns to step ST1000 and repeats the processing of step ST1000 and subsequent steps. In step ST1200, the processor 52 executes the model switching processing of switching the concentration prediction models 96 in accordance with the next step.

In step ST1500, in a case in which there is no next step (NO in step ST1500), the monitoring processing ends.

In step ST1000, the step to be started is determined based on the step name input by the operator, but the processor 52 may determine the step to be started regardless of the input of the operator. For example, the processor 52 can determine the next step to be started by the processor 52 without the input of the operator by detecting a remaining amount of the culture supernatant liquid 17 to be introduced into the immunoaffinity chromatography device 25 through a liquid level sensor or the like.

As described above, the quality monitoring apparatus 41 according to the technology of the present disclosure comprises the processor 52, and the processor 52 executes the monitoring processing of monitoring the quality by using the plurality of concentration prediction models 96A to 96C (an example of a plurality of state prediction models) that predict the antibody concentration Dp (an example of the state of the liquid) related to the quality of the third process 12 as an example of the manufacturing process, using the spectrum measurement data 71 representing the Raman spectrum (an example of spectroscopic spectrum) acquired inline from the liquid produced in the manufacturing process of the biopharmaceutical from the liquid as the input data. Then, the processor 52 can execute the model switching processing of switching the concentration prediction models 96A to 96C during the monitoring processing based on the step identification information (an example of step management information) for managing at least one step included in the third process 12. Further, the plurality of concentration prediction models 96A to 96C have been trained before the monitoring processing is started.

As a result, the quality of the manufacturing process of the biopharmaceutical can be appropriately managed as compared with the related art. That is, in the related art, since the collection of the training data and the update of the state prediction model are performed during the operation of the manufacturing process, in a case in which the state fluctuation is large between the steps, there is a concern that the update of the state prediction model may not be completed in time, and, in this case, the quality management may be insufficient. According to the technology of the present disclosure, the model switching processing is executed during the monitoring processing using the plurality of trained concentration prediction models 96A to 96C before the monitoring processing is started, so that the model switching is quickly performed even in a case in which the fluctuation in the state is large between the steps. As a result, the quality of the manufacturing process of the biopharmaceuticals can be appropriately managed as compared with the related art.

The operator can check the temporal change in the absorbance 64 and the antibody concentration Dp in the third process 12 through the monitoring screen 91. Since the absorbance 64 and the antibody concentration Dp are displayed in real time in time series, it is possible to quickly ascertain whether the third process 12 is being normally performed.

In addition, in the embodiment described above, the model switching processing is processing of switching the concentration prediction model 96 based on the step name (an example of step identification information) before one step included in the third process 12 (an example of a manufacturing process) is started. Therefore, it is possible to use an appropriate state prediction model for each step.

The state prediction model may be switched based on the usage device instead of the step name. The usage device is, for example, the column of each of the chromatography devices 25 to 27.

In addition, in the embodiment described above, the Raman spectrum is used as the spectroscopic spectrum. Since the prediction of the antibody concentration Dp by the Raman spectrum is performed by a known prediction method, reliable and stable prediction accuracy can be expected as compared with a case in which a novel prediction method is used.

In addition, the spectroscopic spectrum may be any of an infrared spectrum or a fluorescence spectrum in addition to the Raman spectrum. Since this is also a known prediction method, reliable and stable prediction accuracy can be expected.

In addition, a plurality of different types of spectroscopic spectra, such as the Raman spectrum and the infrared spectrum, may be used. For example, a method of using the spectroscopic spectrum for each state prediction model is considered.

In addition, in the embodiment described above, the processor 52 outputs the prediction value of the antibody concentration Dp (an example of a state of the liquid) at the preset first time interval based on the spectrum measurement data 71 representing the Raman spectrum (an example of a spectroscopic spectrum). Therefore, it is possible to monitor the fluctuation in the liquid state in real time.

In the embodiment described above, the first time interval is 1 second. In this way, by setting the first time interval to 5 seconds or less and more preferably 1 second or less, it is possible to ascertain the transition of the state of the liquid that fluctuates in a short time.

In addition, in the embodiment described above, the manufacturing process 2 includes the third process 12 that is an example of a purification process of the antibody 19 (an example of a target component) contained in the liquid. The state of the liquid is the concentration of the antibody 19 contained in the liquid. In a case in which the third process 12 is the purification process of the antibody 19, the concentration of the antibody 19 is an important indicator related to the quality. Therefore, the quality can be appropriately monitored as compared with a case in which the concentration of the antibody 19 is not monitored.

In addition, in the embodiment described above, the target component is the antibody 19. Since the antibody 19 is the active ingredient of the biopharmaceutical, the quality of the biopharmaceutical can be appropriately monitored.

In addition, in the embodiment described above, the state of the liquid to be predicted is a state of a protein included in the liquid. Since the protein is a main component contained in the liquid produced in the manufacturing process of the biopharmaceutical, the quality of the biopharmaceutical can be appropriately monitored by monitoring the state of the protein.

### (Method of Acquiring Final Antibody Concentration Dpf)

As shown in FIG. 11 as an example, the final antibody concentration Dpf, which is an example of the final quality, is acquired offline after the completion of the step. The immunoaffinity chromatography step will be described as an example. In a case in which the immunoaffinity chromatography step is completed, the total amount of the first purified liquid 28 purified in the step is accommodated in the recovery container T. The first purified liquid 28 is sampled from the recovery container T, the sampled first purified liquid 28 is analyzed offline, and the final antibody concentration Dpf is acquired. In the offline analysis, for example, the final antibody concentration Dpf is acquired using a method such as high performance liquid chromatography (HPLC).

### (Prediction of Plurality Of Items)

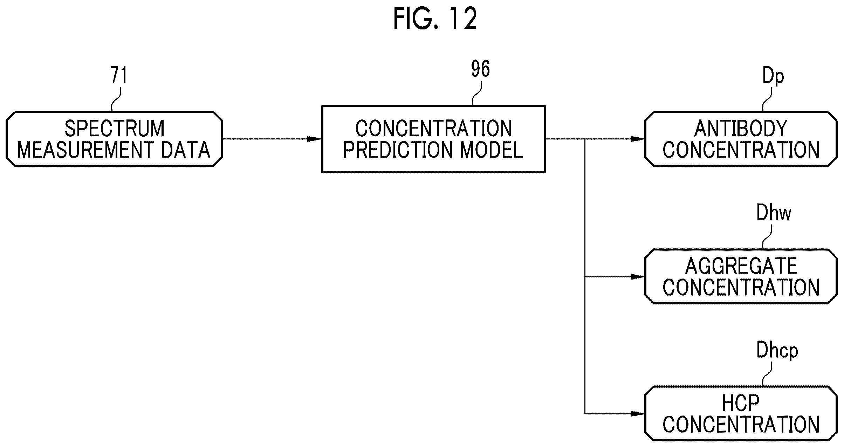

As shown in FIG. 12 as an example, a plurality of items may be predicted by the concentration prediction model 96 based on the spectrum measurement data 71. The plurality of items include an aggregate concentration Dhw, a host cell protein (HCP) concentration Dhcp, and the like, in addition to the antibody concentration Dp. Since the Raman spectrum includes information other than the antibody 19, it is possible to predict the plurality of items.

### (Display Items on Monitoring Screen)

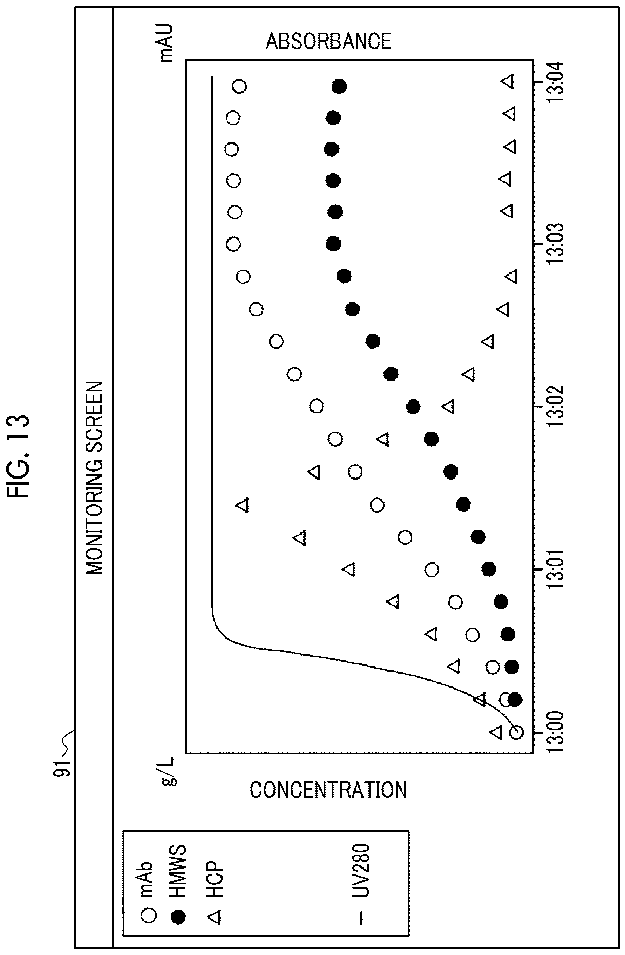

Then, as shown in FIG. 13 as an example, the plurality of predicted items may be displayed on the monitoring screen 91. In this case, it is possible to display the temporal change in the aggregate concentration Dhw and the HCP concentration Dhcp in real time, similarly to the antibody concentration Dp.

FIG. 13 shows the temporal change in the antibody concentration Dp and the like in the elution step, as in FIG. 9. The aggregate and the HCP are impurities. In the elution step, since the HCP is considered to be adsorbed non-specifically to the column, the elution is faster than that of the antibody 19. On the other hand, since the aggregate 21 (indicated by HMWS in FIG. 13) is a collection of the antibodies 19, the elution proceeds in substantially the same manner as the antibody 19. FIG. 13 shows such a state.

### (Model Switching in Sub-Step)

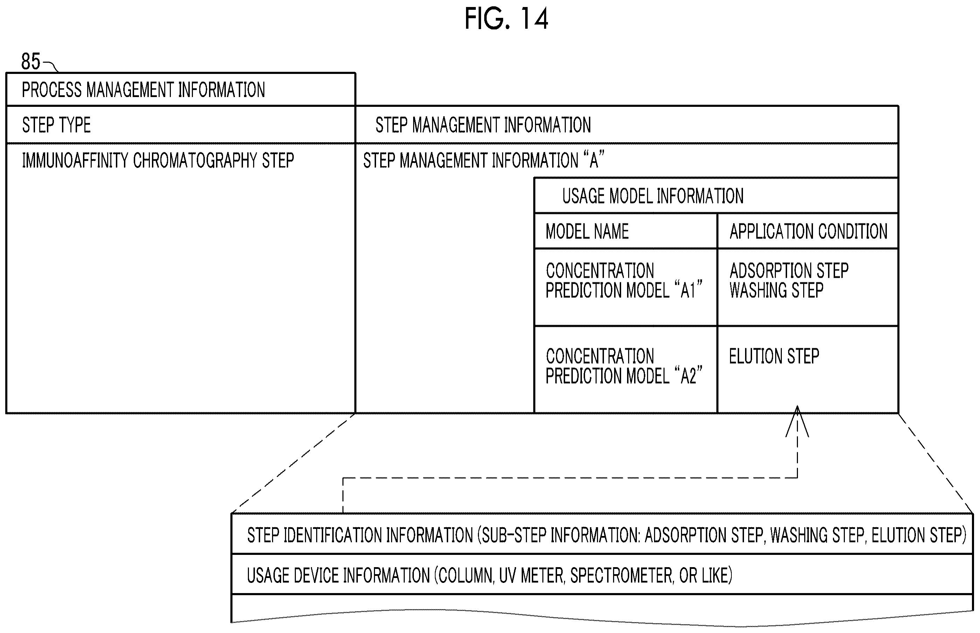

As in modification examples of the first embodiment shown in FIGS. 14 to 16 as an example, the concentration prediction models 96 may be switched between a plurality of sub-steps in one step included in the third process 12. In the examples shown in FIGS. 14 to 16, in the three sub-steps of the adsorption step, the washing step, and the elution step in the immunoaffinity chromatography step, a concentration prediction model 96A1 is used in the adsorption step and the washing step, and a concentration prediction model 96A2 is used in the elution step.

As shown in FIG. 14, in the step management information of the immunoaffinity chromatography step of the process management information 85, as the usage model information, a model name of the concentration prediction model 96A1 (a name in the step management information is a concentration prediction model "A1") and a model name of the concentration prediction model 96A2 (a name in the step management information is a concentration prediction model "A2") are described. Then, the adsorption step and the washing step are described as application conditions of the concentration prediction model 96A1, and the elution step is described as an application condition of the concentration prediction model 96A2. In addition, the step identification information describes the order of the adsorption step, the washing step, and the elution step as sub-step information. The processor 52 executes the model switching processing of switching the two concentration prediction models 96A1 and 96A2 based on the description of the sub-step and the application condition included in the step identification information.

As described above, the adsorption step is a step of introducing the culture supernatant liquid 17 into the immunoaffinity chromatography device 25 to adsorb the antibody 19 to the column, and the washing step is a step of washing away impurities, which are non-specifically adsorbed, other than the antibody 19 that is the specifically adsorbed to the column by introducing the washing solution. Therefore, in the adsorption step and the washing step, the antibody concentration Dp of the liquid flowing out from the immunoaffinity chromatography device 25 is very low. On the other hand, the elution step is a step of introducing the eluent into the column to elute the antibody 19 specifically adsorbed to the column, and the antibody concentration Dp of the liquid flowing out from the immunoaffinity chromatography device 25 is increased. As described above, the fluctuation in the antibody concentration Dp is large in the adsorption step and the washing step, and the elution step. In addition, in the adsorption step and the washing step, and the elution step, the liquid to be introduced into the immunoaffinity chromatography device 25 is changed, and thus the components of the liquid that flows out are also largely changed in many cases.

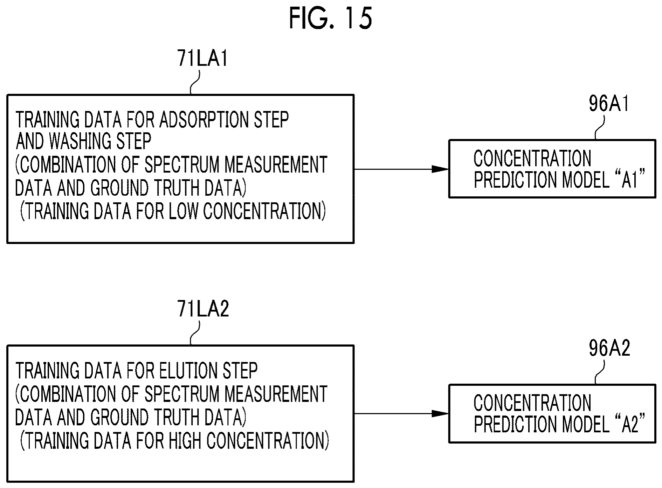

As shown in FIG. 15 as an example, the concentration prediction model 96A1 is a trained model that has been trained using training data 71LA1 for the adsorption step and the washing step. The concentration prediction model 96A2 is a trained model that has been trained using training data 71LA2 for the elution step. The training data 71LA1 is training data for a relatively low concentration and is training data suitable for the components of the liquid to be measured in the adsorption step and the washing step. The training data 71LA2 is training data for a relatively high concentration and is training data suitable for the components of the liquid to be measured in the elution step. Therefore, the prediction accuracy of the concentration prediction model 96A1 is high in a low concentration range, and the prediction accuracy of the concentration prediction model 96A2 is high in a high concentration range.

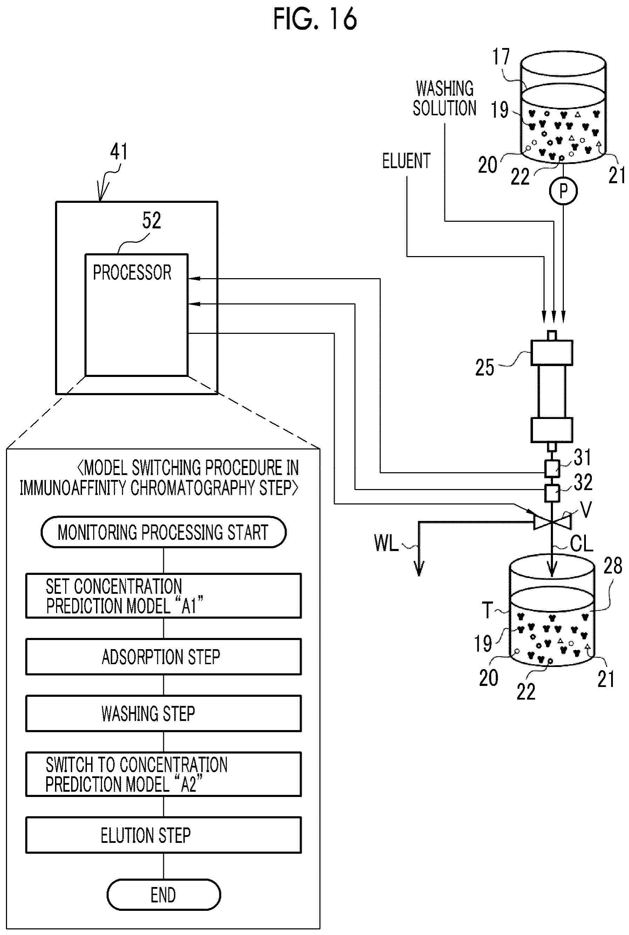

FIG. 16 is an example of a model switching procedure in the immunoaffinity chromatography step. As shown in FIG. 16, in a case of starting the monitoring processing of the immunoaffinity chromatography step, the processor 52 first determines that a first sub-step of the immunoaffinity chromatography step is the adsorption step with reference to the step identification information in the step management information. Then, the processor 52 sets the concentration prediction model 96A1 for the low concentration with reference to the usage model information in the step management information. In the adsorption step and the washing step, the prediction of the antibody concentration Dp using the concentration prediction model 96A1 is performed, and the temporal change in the predicted antibody concentration Dp is displayed on the monitoring screen 91 in real time.

Then, after the adsorption step and the washing step are completed and before starting the elution step, the processor 52 switches from the concentration prediction model 96A1 for the low concentration to the concentration prediction model 96A2 for the high concentration. In the elution step, the prediction of the antibody concentration Dp using the concentration prediction model 96A2 is performed, and the temporal change in the predicted antibody concentration Dp is displayed in real time on the monitoring screen 91.

As described above, the model switching processing also includes processing of switching the state prediction model based on the step identification information before the sub-step is started. In the examples shown in FIGS. 14 to 16, the concentration prediction model 96A1 for the low concentration and the concentration prediction model 96A2 for the high concentration are used separately during the monitoring processing, so that appropriate quality monitoring can be performed as compared with the related art.

Even in the processing procedure shown in FIG. 16, as shown in step ST1000 of FIG. 10, the processor 52 determines the sub-step to be started, based on the input of the step name by the operator. Alternatively, the processor 52 may determine the sub-step to be started, regardless of the input of the step name by the operator. For example, the processor 52 determines that the washing step is completed, by detecting the remaining amount of the washing solution. Then, the step to be started next can be determined as the elution step based on the step identification information.

### [Second Embodiment]

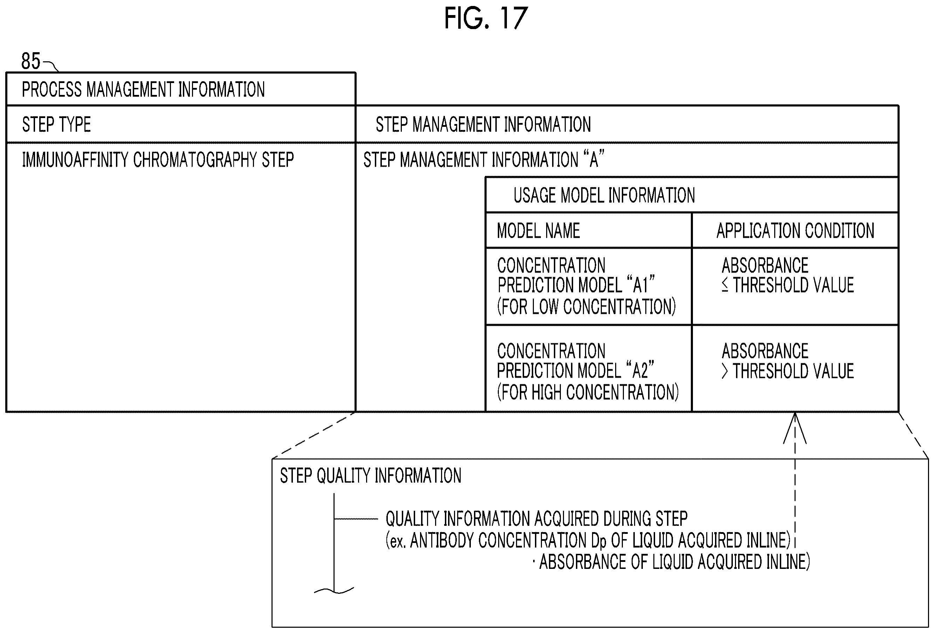

As an example, the model switching processing of the second embodiment shown in FIGS. 17 to 19 has an aspect in which the concentration prediction models 96 are switched based on the quality information that is one of the step quality information and that is acquired during the step. The first embodiment and the second embodiment are different in that the concentration prediction models 96 are switched before the start of the step based on the step identification information in the first embodiment, whereas the concentration prediction models 96 are switched based on the quality information acquired during the step after the start of the step in the second embodiment.

As described in the usage model information of the step management information shown in FIG. 17, the second embodiment is the same as the modification example of the first embodiment shown in FIG. 14 in that two models, that is, the concentration prediction model 96A1 for the low concentration and the concentration prediction model 96A2 for the high concentration, are selectively used. However, the second embodiment is different from the modification example of the first embodiment in that, in the usage model information, the application condition of each of the concentration prediction models 96A1 and 96A2 is defined by a relationship between the absorbance 64A and a preset threshold value, instead of the step name. The absorbance 64A is the absorbance of the antibody 19 and the aggregate 21 in the liquid, which is acquired inline by the UV detector 31. The absorbance 64A is stored in the storage 45 as time-series data, but is also used as the step quality information of the process management information 85.

In the second embodiment, the concentration prediction model 96A1 for the low concentration and the concentration prediction model 96A2 for the high concentration are used as in the first embodiment, but the concentration prediction model 96A that has been trained using training data more suitable for the second embodiment may be used.

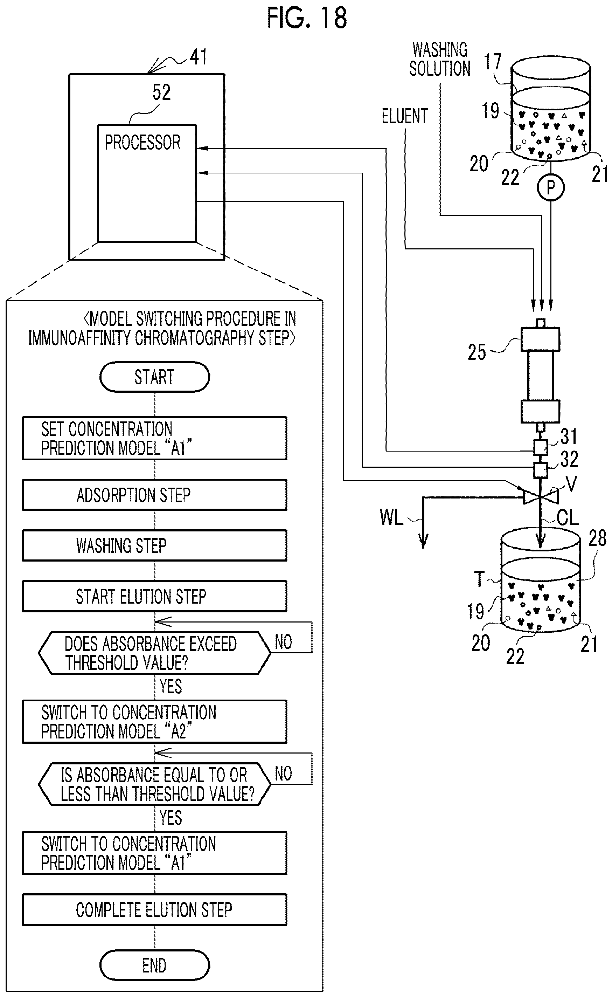

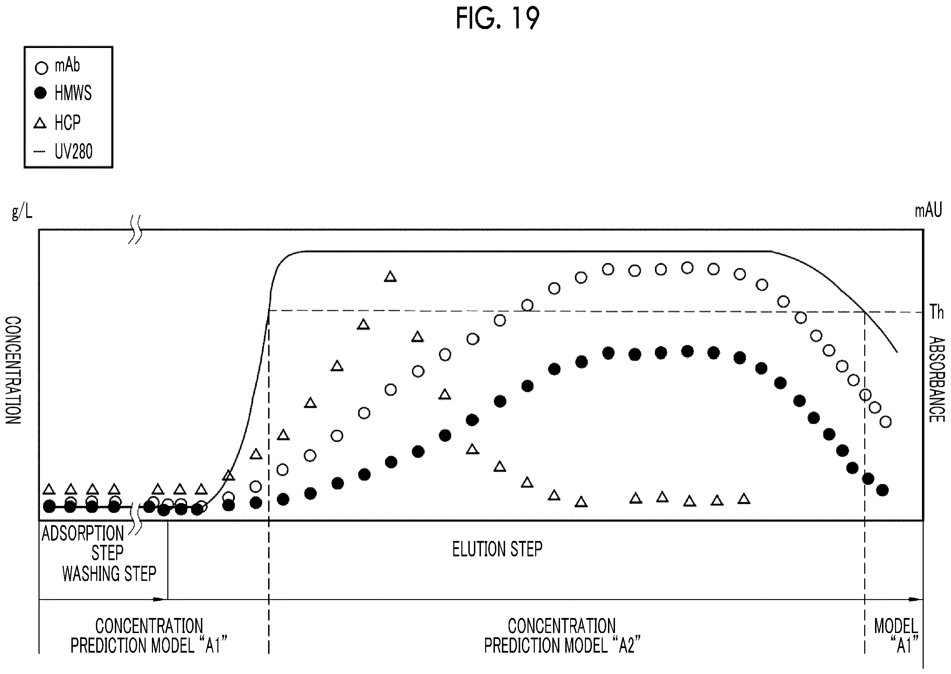

As shown in FIGS. 18 and 19, the processor 52 starts the elution step in a state in which the concentration prediction model 96A1 for the low concentration used in the adsorption step and the washing step is set. The processor 52 predicts the antibody concentration Dp using the concentration prediction model 96A1 for the low concentration, and displays the temporal change in the antibody concentration Dp on the monitoring screen 91. After starting the elution step, the processor 52 collates the absorbance 64 that changes over time with the threshold value during the step. Then, the concentration prediction model 96A1 for the low concentration is used while the absorbance 64 is equal to or less than the threshold value.

As shown in FIG. 19, the threshold value (indicated by Th in FIG. 19) is set to, for example, a value before the absorbance 64 is saturated. As described above, the detection light of the UV detector 31 is also sensitive to impurities including the aggregate 21 and the HCP other than the antibody 19, and thus the absorbance 64 is saturated before the antibody concentration Dp shows a peak.

At the beginning of the elution step, the concentration of the antibody concentration Dp is low. The elution of the antibody 19 from the column eventually progresses, and the antibody concentration Dp starts to rapidly increase. In a case in which the absorbance 64A exceeds the threshold value, the processor 52 performs the switching to the concentration prediction model 96A2 for the high concentration. The processor 52 predicts the antibody concentration Dp using the concentration prediction model 96A2 for the high concentration, and displays the temporal change in the antibody concentration Dp on the monitoring screen 91. The processor 52 further continues the collation between the absorbance 64A and the threshold value, and, in a case in which the absorbance 64A is equal to or less than the threshold value, performs the switching to the concentration prediction model 96A1 for the low concentration again. Thereafter, the concentration prediction model 96A1 is used until the elution step is completed.

As described above, in the example shown in FIG. 19, the elution step starts with the concentration prediction model 96A1 for the low concentration. Then, in the elution step, the switching to the concentration prediction model 96A2 for the high concentration is performed before the absorbance 64A is saturated in accordance with the change in the absorbance 64A, and, in a case in which the absorbance 64 decreases due to the decrease in the antibody concentration Dp, the switching to the concentration prediction model 96A1 for the low concentration is performed again.

The absorbance 64A is an example of "quality information acquired during the step". Based on such quality information, the concentration prediction models 96A, which is an example of the state prediction models, are switched, so that an appropriate concentration prediction model 96A can be used even in a case in which the step quality fluctuates during the step.

In addition, the absorbance 64A is information that is related to the concentration of the antibody 19 in the liquid and that is acquired inline in the immunoaffinity chromatography step, and is an example of "information that is related to the concentration of the target component in the liquid and that is acquired inline in the step". The manufacturing process 2 includes the third process 12 that is the purification process of the target component contained in the liquid. The state of the liquid predicted by the concentration prediction model 96A is the concentration of the antibody 19 that is the target component contained in the liquid. In a case of predicting the antibody concentration Dp, the absorbance 64A, which is information related to the concentration of the antibody 19, is one of the important indicators related to the quality of the step, so that it is possible to select an appropriate model by switching the concentration prediction models 96A based on the absorbance 64A.

### (Modification Example of Second Embodiment)

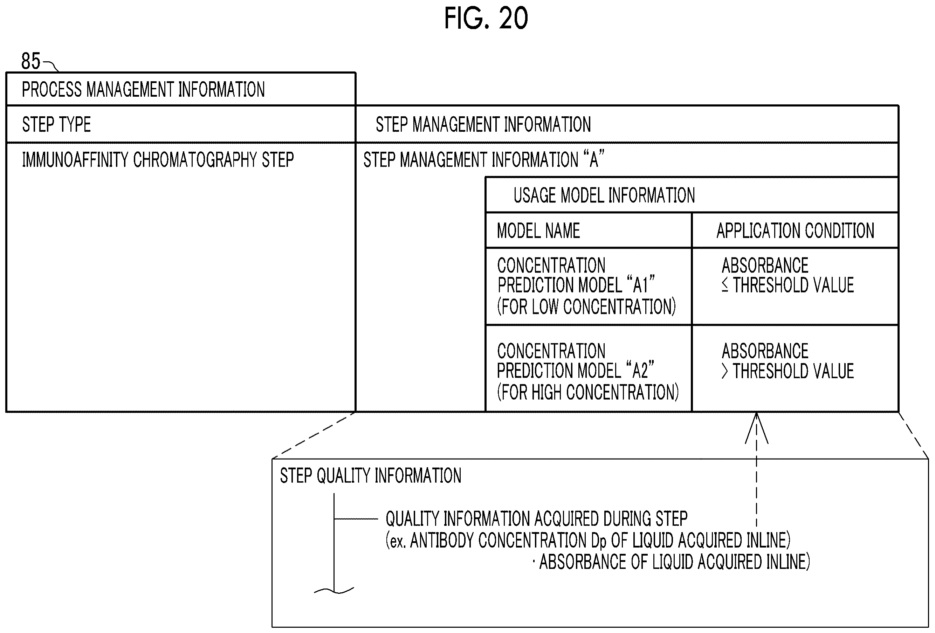

Since modification examples of the second embodiment shown in FIGS. 20 to 22 are substantially the same as the second embodiment shown in FIGS. 17 to 19, only the differences will be described. The difference is that, in the second embodiment shown in FIGS. 17 to 19, the absorbance 64A, which is the measurement value of the UV detector 31, is used as the "information that is related to the concentration of the target component of the liquid and that is acquired inline in the step", whereas in the modification examples shown in FIGS. 20 to 22, the antibody concentration Dp, which is the prediction value by the concentration prediction model 96A, is used.

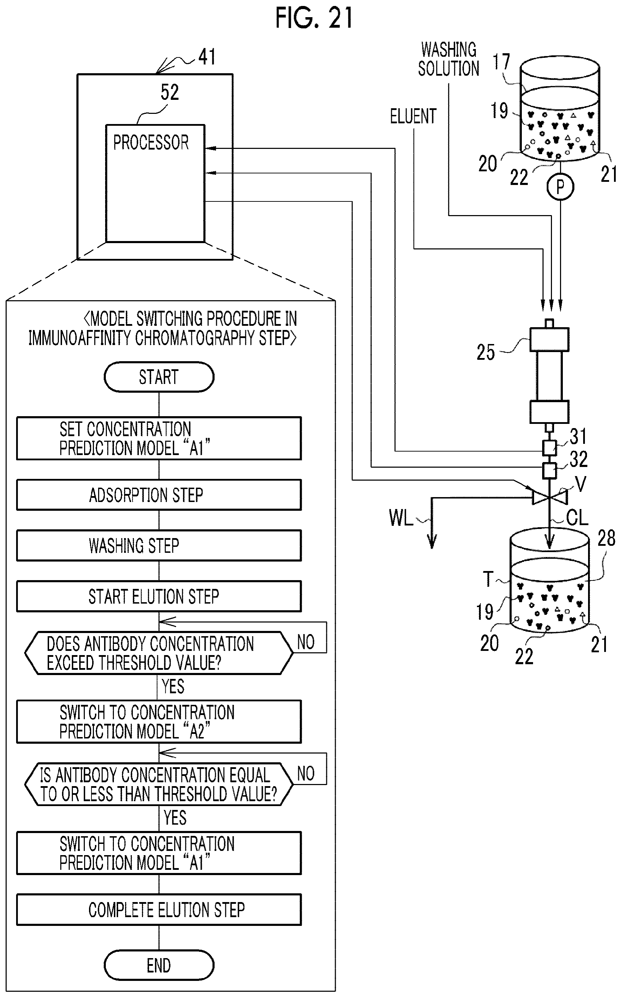

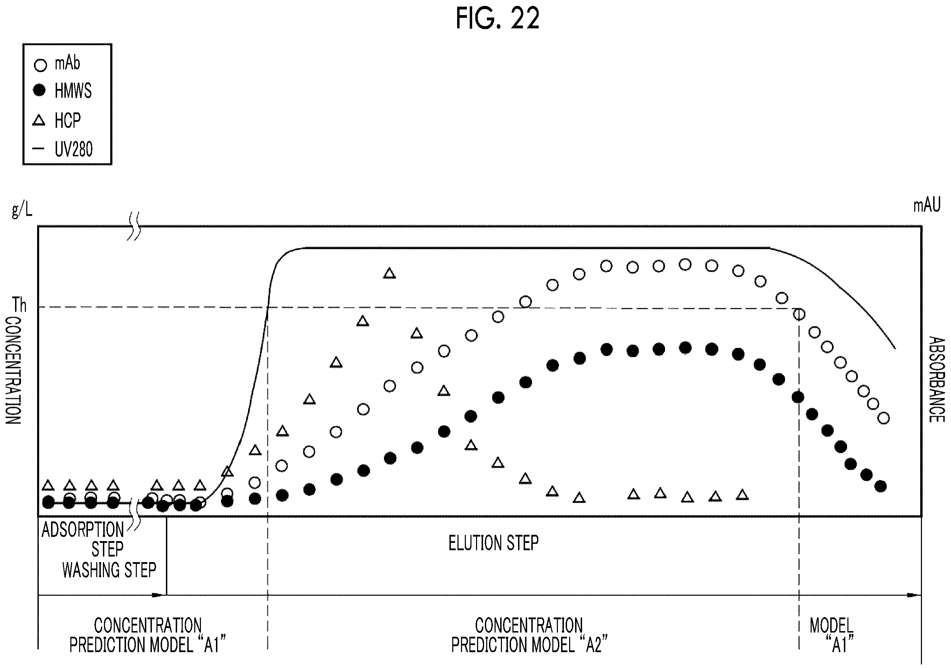

As shown in FIG. 20, in the usage model information of the step management information, a relationship between the antibody concentration Dp and the threshold value is defined as the application condition. As shown in FIGS. 21 and 22, after the start of the elution step, the processor 52 collates the antibody concentration Dp that changes over time with the threshold value during the step. Then, the concentration prediction model 96A1 for the low concentration is used while the antibody concentration Dp is equal to or less than the threshold value.

In a case in which the antibody concentration Dp predicted by the concentration prediction model 96A1 for the low concentration exceeds the threshold value, the processor 52 performs the switching to the concentration prediction model 96A2 for the high concentration. The processor 52 further continues the collation between the antibody concentration Dp and the threshold value, and, in a case in which the antibody concentration Dp is equal to or less than the threshold value, performs the switching to the concentration prediction model 96A1 for the low concentration again. Thereafter, the concentration prediction model 96A1 is used until the elution step is completed.

As described above, in a case of predicting the antibody concentration Dp, the prediction value of the antibody concentration Dp is one of the important indicators related to the step quality, as in the absorbance 64A, so that it is possible to select an appropriate model by switching the concentration prediction models 96A based on the antibody concentration Dp.

In addition, as can be seen by comparing FIG. 19 with FIG. 22, regarding the "information that is related to the concentration of the target component of the liquid and that is acquired inline in the step", either the absorbance 64 or the antibody concentration Dp can be used to obtain similar switching timing, depending on how the threshold value is set.

### (Other Modification Example 1: Method of Obtaining Final Antibody Concentration Dpf)

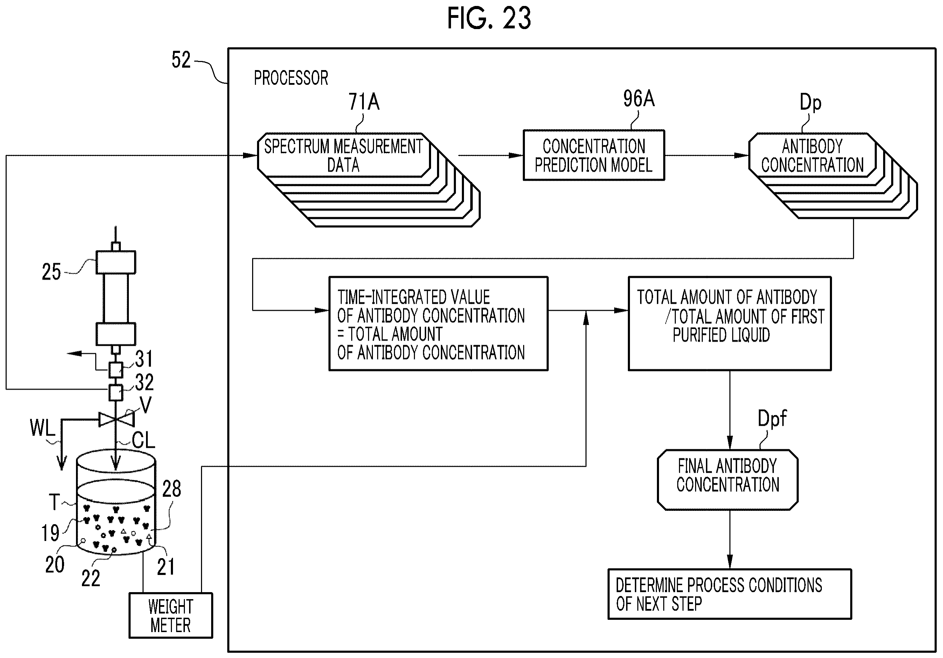

As shown in FIG. 23 as an example, the final antibody concentration Dpf can be obtained from the antibody concentration Dp that is the prediction value of the concentration prediction model 96A. For example, in the immunoaffinity chromatography step, the processor 52 predicts the antibody concentration Dp at a regular period using the concentration prediction model 96A based on the spectrum measurement data 71A acquired at a regular period. The processor 52 integrates the predicted antibody concentration Dp in a time direction. The time-integrated value of the antibody concentration Dp is an estimated value of the total amount of the antibody 19 contained in the total amount of the first purified liquid 28 that is finally recovered. It is possible to derive the final antibody concentration Dpf by dividing the total amount of the antibody 19 by the total amount of the first purified liquid 28. It is possible to acquire the total amount of the first purified liquid 28 using, for example, a weight meter.

The total amount of the first purified liquid 28 can also be acquired by measuring a liquid level of the first purified liquid 28 that is finally recovered in the recovery container T by the liquid level sensor provided in the recovery container T. Alternatively, it is possible to determine the total amount of the first purified liquid 28 using a flowmeter installed in the recovery line CL. Specifically, a flow rate of the first purified liquid 28 that passes through the recovery line CL during the period of the elution step of the immunoaffinity chromatography step is measured, and the time-integrated value of this flow rate is calculated. In addition, the total amount of the first purified liquid 28 can also be calculated from a rotation speed of the pump P during the period of the elution step.

Although the final antibody concentration Dpf obtained by the method shown in FIG. 23 is the estimated value, the final antibody concentration Dpf can be obtained immediately after the immunoaffinity chromatography step is completed without performing the offline analysis as shown in FIG. 11. In addition, an intermediate progress of the final antibody concentration Dpf may be calculated in real time during the elution step and displayed on the monitoring screen 91 in real time. In addition, it is also possible to obtain a recovery rate of the antibody 19 and the like based on the intermediate progress of the final antibody concentration Dpf, and it is also possible to determine the switching timing of the valve V for finishing the elution step based on such a value.

As shown in FIG. 23, the final antibody concentration Dpf obtained immediately after the completion of the step can be used, for example, for determining process conditions of the next step. As the process conditions, there are various conditions that can be set in a case of carrying out the step, such as setting of the usage device used and parameters. The process conditions of the next step are specifically as follows. In the present example, the next step after the immunoaffinity chromatography step is the cation exchange chromatography step. In the present example, in the cation exchange chromatography device 26 used in the cation exchange chromatography step, the amount of the antibody 19 that can be added is set in accordance with the size of the column and the properties of the resin filled in the column. Therefore, in a case in which the final antibody concentration Dpf can be grasped immediately after the completion of the immunoaffinity chromatography step, the amount of the first purified liquid 28 added to the column of the cation exchange chromatography device 26 of the next step can be determined based on the final antibody concentration Dpf.

The final antibody concentration Dpf obtained based on the prediction value of the concentration prediction model 96A is an example of a "final quality of the step, indicating a final state of an entire liquid produced in the step" according to the technology of the present disclosure. By obtaining the final quality of the step based on the prediction value in this way, it is possible to use the final quality of the previous step for determining the process conditions of the next step even in a case in which the time interval between the steps is short.

Moreover, according to the technology of the present disclosure, since the state prediction model is appropriately switched in accordance with the step management information during the monitoring processing of the manufacturing process 2, the prediction accuracy of the estimated value of the final quality is also improved. Therefore, the process conditions of the next step can be appropriately determined.

### (Other Modification Example 2)

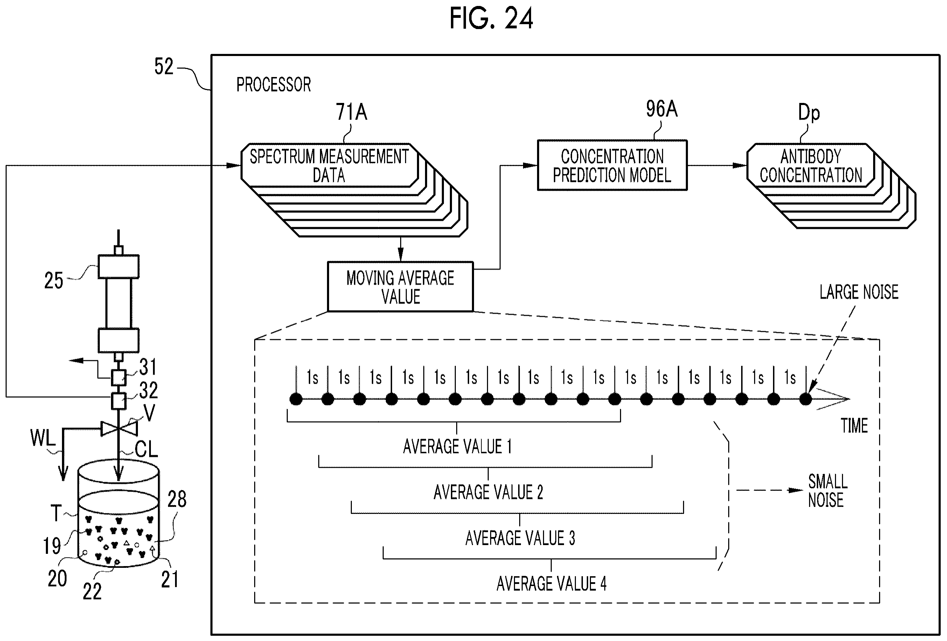

In addition, as an example, as shown in FIG. 24, the processor 52 may acquire the spectrum measurement data 71 (an example of a spectroscopic spectrum) at the second time interval equal to or shorter than the first time interval, and output the antibody concentration Dp (an example of a prediction value of the state of the liquid) based on a moving average value of a plurality of the acquired spectrum measurement data 71.

As shown in FIG. 24, the processor 52 derives, for example, the moving average value of the spectrum measurement data 71 of 10 times acquired at the second time interval (in this example, 1 second). Average values 1, 2, 3, 4, and the like are moving average values calculated by shifting the spectrum measurement data 71 to be averaged by one time. The processor 52 outputs the antibody concentration Dp, which is an example of a prediction value of the state of the liquid, based on the moving average value of the spectrum measurement data 71.

Noise of one measurement value of the spectrum measurement data 71 is large. By averaging the data of 10 times noise can be suppressed. Then, the acquisition interval can be shortened by using the moving average value. In a case in which noise of the data used for prediction is suppressed, the prediction accuracy is also improved.

As described above, since the processor 52 outputs the moving average value of the spectroscopic spectrum used for prediction, it is possible to perform prediction with high accuracy in which noise is suppressed, at a short time interval.

The reason why the quality monitoring apparatus 41 uses the trained state prediction model before the monitoring processing is started is to enable the switching of the state prediction models during the monitoring processing. That is, as described in the embodiment described above, the quality monitoring apparatus 41 assumes that the prediction is repeated at a relatively short time interval, such as real-time prediction of the antibody concentration Dp in the step. Therefore, using the moving average value of the spectroscopic spectrum in the prediction is a technology for aiming at improving the prediction accuracy in the prediction with a short time interval, and a synergistic effect can be expected in relation to the model switching processing according to the technology of the present disclosure.

### (Example 1)

An example of a form in which the modification example of the first embodiment shown in FIGS. 14 to 16, the modification example of FIG. 23 in which the process conditions of the next step are determined based on the final antibody concentration Dpf, and the modification example of FIG. 24 in which the moving average value is output are combined is shown as Example 1.

In the present example, as described in the embodiment described above, the culture supernatant liquid 17 was a culture supernatant liquid of the CHO cell, and a protein A column (product name: MabSelect SuRE, manufactured by Cytiva) was used as the column of the immunoaffinity chromatography device 25. The UV detector 31 and the Raman spectrometer 32 shown in the above-described embodiment were installed downstream of the column. The measurement conditions of the Raman spectrum during the immunoaffinity chromatography step were as described in the above-described embodiment in which the laser output was 500 mW, the laser wavelength was 785 nm, and the exposure time was 1 second. In addition, as shown in FIG. 24, in the prediction of the concentration prediction model 96A, the moving average value of the spectrum measurement data 71 acquired at the interval of 1 second for 10 times was used. The model switching processing was performed based on the step name of the sub-step as shown in FIG. 14. The switching of the valve V was performed based on whether the absorbance 64A exceeded the preset threshold value of 1000 mAU. Furthermore, in order to calculate the final antibody concentration Dpf immediately after the completion of the step, the flowmeter was installed in the recovery line CL.

The immunoaffinity chromatography step was started in a state in which the concentration prediction model 96A1 for the low concentration was set. Further, the monitoring processing was started, and the display of the temporal change in the antibody concentration Dp was also started. In a case in which the step entered the elution step, the quality monitoring apparatus 41 performed the switching to the concentration prediction model 96A2 for the high concentration corresponding to the step name with reference to the step management information.

Then, the immunoaffinity chromatography device 25 switched the eluent to be introduced into the column to an acidic buffer liquid. The antibody 19 adsorbed to the column started to elute by the acidic buffer liquid and started to flow to the downstream side of the column. The absorbance 64A measured by the UV detector 31 started to increase after a while from the start of the elution step. At the same time, the antibody concentration Dp, which is the prediction value, also started to increase. Since the absorbance 64A exceeded 1000 mAU, which is the threshold value set in advance, the valve V was operated, and the flow passage of the liquid from the immunoaffinity chromatography device 25 was switched from the waste liquid line WL to the recovery line CL. From this point, the recording of the antibody concentration Dp was started. In a case in which most of the antibody 19 adsorbed to the column flows down, the absorbance 64A gradually started to decrease, and in a case in which the absorbance 64A was less than the threshold value of 1000 mAU, the valve V was operated, and the switching from the recovery line CL to the waste liquid line WL was performed. At this point, the recording of the flow rate of the first purified liquid 28 and the antibody concentration Dp, which passed through the recovery line CL, was completed.

In a case in which the recording was completed, the processor 52 calculated the time-integrated value of the antibody concentration Dp to obtain the total amount of the antibody 19 in the same manner as in a case shown in FIG. 23. In addition, the processor 52 calculated the total amount of the first purified liquid 28 based on the time-integrated value of the flow rate measured by the flowmeter, and divided the total amount of the antibody 10 by the first purified liquid 28 to obtain the final antibody concentration Dpf. The final antibody concentration Dpf was 23.9 g/L. Although this value was the estimated value, an actually measured value of the final antibody concentration Dpf was measured by offline analysis, and was confirmed to be 24.1 g/L. A value close to the actually measured value was obtained as the final antibody concentration Dpf of the estimated value.

As a comparative example, a simulation of the prediction value in a case in which the model switching was not performed in the immunoaffinity chromatography step was performed. Specifically, the concentration prediction model 96A was trained using the training data 71LA in which the spectrum measurement data 71A having different ranges of the antibody concentration Dp, that is, the spectrum measurement data 71A acquired in the adsorption step and the washing step and the spectrum measurement data 71A acquired in the elution step were mixed without distinction. Then, the antibody concentration Dp was predicted by using the concentration prediction model 96A, and the final antibody concentration Dpf was obtained by the method shown in FIG. 23. In the simulation of the comparative examples, the final antibody concentration Dpf was 23.4 g/L.

As described above, a difference between the prediction value and the actually measured value of the final antibody concentration Dpf of Example 1, in which the model for the low concentration and the model for the high concentration were switched in accordance with the step name, was 0.2 g/L (24.1 - 23.9). On the other hand, in the simulation of Comparative Example, the difference between the prediction value and the actually measured value of the final antibody concentration Dpf was 0.7 g/L (24.1 - 23.4), and the prediction accuracy was lower than that of Example 1. As a result, it was possible to confirm the effect of the model switching.

In addition, the recovered first purified liquid 28 was neutralized with Tris Hydrochloride Acid Buffer (Tris-HCL) in order to add the first purified liquid 28 to the cation exchange chromatography of the next step. In this case, the final antibody concentration Dpf was diluted and decreased, but in a case in which the final antibody concentration Dpf after the neutralization was calculated from an amount of the neutralizing liquid added, the final antibody concentration Dpf was 22.8 g/L. Then, the neutralized first purified liquid 28 was introduced into the cation exchange chromatography device 26. A column adsorption amount of the cation exchange chromatography was 50 g/L Resin. Immediately after the completion of the previous step, the final antibody concentration Dpf of the first purified liquid 28 was determined, and thus it was possible to immediately calculate and determine that the neutralized first purified liquid 28 of 2.19 mL/L Resin was required to be added in the next step, and under the process conditions, the cation exchange chromatography step of the next step could be started. As described above, since the prediction accuracy of the final antibody concentration Dpf is improved by performing the model switching in Example 1, the process conditions of the next step can be appropriately determined as compared with Comparative Example 1 in which the prediction accuracy is low.

### (Example 2)

Example 2 is an example corresponding to FIGS. 17 to 19 in which the concentration prediction models 96A to be used are automatically switched based on the absorbance 64A by the UV detector 31.

In Example 2, in the immunoaffinity chromatography step, the threshold value of the absorbance 64 used for the model switching point was set to 2500 mAU at which the absorbance 64 by the UV detector 31 started to be saturated. As the application conditions, the concentration prediction model 96A1 for the low concentration was used for "absorbance 64A ≤ 2500 mAU", and the concentration prediction model 96A2 for the high concentration was used for "absorbance 64A > 2500 mAU". The concentration prediction model 96A1 for the low concentration was a model trained using the training data of the antibody concentration Dp in a range of 0 to 7 g/L. As the concentration prediction model 96A2 for the high concentration, a model trained using training data of the antibody concentration Dp in a range of 0 to 25 g/L was prepared. As described above, the training data of the concentration prediction model 96A2 for the high concentration includes the training data in which the antibody concentration Dp is high, which is not included in the training data of the concentration prediction model 96A1 for the low concentration.

The elution step was performed while fractionating the eluted first purified liquid 28 using a fraction collector. In the elution step, since the absorbance 64A showed a trend of rising to about 3000 mAU, then saturating, and then eventually decreasing to 0 mAU after saturation, the model switching processing was executed in the order of the concentration prediction model 96A1 for the low concentration, the concentration prediction model 96A2 for the high concentration, and the concentration prediction model 96A1 for the low concentration. After the completion of the elution step, the first purified liquid 28 fractionated by the fraction collector was analyzed offline, the prediction accuracy was evaluated using the analysis value, and the RMSE was 0.30. On the other hand, as in the related art, in a case in which only the concentration prediction model 96A2 for the high concentration was used in the elution step, the RMSE is 0.39, and the prediction accuracy was improved by about 25%. In addition, in a case in which only the prediction value in the low concentration range of the absorbance 64A ≤ 2500 mAU was focused, the RMSE of the concentration prediction model 96A1 for the low concentration was 0.16, and the RMSE of the concentration prediction model 96A2 for the high concentration was 0.33 as in the related art, and the prediction accuracy was improved by 50% or more. As a result, it was possible to confirm the effect of the model switching processing.

In the above-described example, the antibody concentration is described as the state of the liquid that is the quality of the manufacturing process 2, but the present disclosure is not limited to this. In addition to the antibody concentration, examples thereof include a viable cell density, a cell status, a glucose concentration, a glutamic acid concentration, an amino-acid concentration, a solvent-component concentration, an additive concentration, a lactate concentration, an ammonia concentration, host-cell protein and nucleic-acid concentrations, other cellular-metabolite concentrations, an antibody aggregate concentration, an antibody fragment concentration, and a charge-variant concentration. Among these, the antibody concentration and the antibody aggregate concentration are preferable, and the antibody concentration is particularly preferable.

In the above-described example, the Raman spectrum is described as the spectroscopic spectrum, but other spectra may be used, such as an infrared spectrum, a near infrared spectrum, an ultraviolet visible absorption spectroscopy (UV-Vis) spectrum, and a fluorescence spectrum. Among these, the Raman spectrum is particularly preferable. The Raman spectrum is likely to reflect information derived from a functional group of the amino acid of the protein. This is to contribute to the prediction accuracy of the concentration of the antibody 19 that is the protein.

The third process 12, which is the purification process, is described as the manufacturing process 2 that is the quality monitoring target, and three steps of the immunoaffinity chromatography step, the cation exchange chromatography step, and the anion exchange chromatography step are described as the steps included in the third process 12, but the present disclosure is not limited thereto. For example, the manufacturing process 2 to be monitored may be a cell culture step (fed-batch culture, perfusion culture) or a cell separation step (centrifugation, filtration separation, or the like) during the manufacture of the biopharmaceutical. Further, as the step included in the purification process, a size exclusion chromatography step, a hydrophobic interaction chromatography step, and the like may be used as the chromatography step. Other examples thereof include a virus inactivation step, a virus filtration step, and a filter filtration step (ultrafiltration membrane and dialysis filtration membrane). Among these, as the manufacturing process 2 that is the quality monitoring target, the chromatography step is preferable, and the immunoaffinity chromatography step and the cation exchange chromatography step are particularly preferable.

Although the machine learning model is described as the state prediction model and the neural network 105 is described as the algorithm of the machine learning model, the present disclosure is not limited to this. The machine learning algorithm may be a random forest, a support vector machine, and the like. In addition, multivariate analysis such as linear regression and logistic regression may be used instead of the machine learning model.

The predicted antibody concentration itself and the absorbance are described as the step quality information of the step management information, but the present disclosure is not limited to this, and, for example, a range of the antibody concentration, a range of the aggregate concentration, and the like may be used. Preferably, the measurement value of the sensor such as the UV detector 31 that measures the absorbance and information calculated based on the actually measured value are preferable.

Although the final quality of the antibody concentration is described as the final quality, the final quality is not limited to this. Examples thereof include a final quality of a viable cell density, a cell status, a glucose concentration, a glutamic acid concentration, an amino-acid concentration, a solvent-component concentration, an additive concentration, a lactate concentration, an ammonia concentration, host-cell protein and nucleic-acid concentrations, other cellular-metabolite concentrations, an antibody aggregate concentration, an antibody fragment concentration, or a charge-variant concentration. Among these, the final qualities of the antibody concentration and the antibody aggregate concentration are preferable, and the final quality of the antibody concentration is particularly preferable.

The acquisition interval of the data acquired as the time-series data in the monitoring processing can be output, for example, every 1 hour, every 1 minute, or every 1 second, but is preferably in a range from every 1 minute to every 1 second, and most preferably every 1 second.

The biopharmaceutical including the antibody 19 is called an antibody drug and is widely used not only for the treatment of chronic diseases, such as cancer, diabetes, and rheumatoid arthritis, but also for the treatment of rare diseases, such as hemophilia and a Crohn's disease. Therefore, in a case in which the antibody 19 is used as the target component, it is possible to promote the development of antibody pharmaceutical widely used for the treatment of various diseases.

It should be noted that the target component is not limited to the antibody 19. The target component may be an aggregate 21, a cytokine, a hormone, and the like. In addition, a cell-derived protein, a cell-derived DNA, and the like may be used as the target component.

In each of the embodiments described above, for example, the following various processors can be used as a hardware structure of a processing unit, such as the processor 52, that executes various types of processing. As described above, the various processors include, in addition to the CPU 47, which is a general-purpose processor that executes software (operation program 75) to function as the various processing units, a programmable logic device (PLD), which is a processor of which a circuit configuration can be changed after the manufacturing, such as a field programmable gate array (FPGA), a dedicated electric circuit, which is a processor having a circuit configuration designed exclusively for executing specific processing, such as an application specific integrated circuit (ASIC), and the like.

One processing unit may be configured by one of these various processors or by a combination of two or more processors of the same type or different types (for example, a combination of a plurality of FPGAs and/or a combination of a CPU and an FPGA). Also, a plurality of processing units may be configured by one processor.

Examples in which the plurality of processing units are configured by one processor include, first, as represented by a computer, such as a client and a server, a form in which one processor is configured by a combination of one or more CPUs and software, and the processor functions as the plurality of processing units. Second, as represented by a system-on-a-chip (SoC) or the like, there is a form in which a processor, which implements the functions of the entire system including the plurality of processing units with a single integrated circuit (IC) chip, is used. In this way, as the hardware structure, the various processing units are configured by one or more of the various processors.

Furthermore, as the hardware structure of the various processors, more specifically, an electric circuit (circuitry) in which circuit elements, such as semiconductor elements, are combined can be used.

It is possible to understand the technology according to the following supplementary notes, based on the above description.

### [Supplementary Note 1]

A quality monitoring apparatus comprising: a processor configured to execute monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and model switching processing of switching the state prediction models during the monitoring processing based on step management information for managing at least one step included in the manufacturing process, in which the plurality of state prediction models have been trained before the monitoring processing is started, and the step management information includes at least one of step identification information for identifying at least one step included in the manufacturing process, usage device information of a usage device used in the step, or step quality information that is related to a quality of each step and that is acquirable in a case in which the step is performed at least once.

### [Supplementary Note 2]

The quality monitoring apparatus according to supplementary note 1, in which the model switching processing includes processing of switching the state prediction models based on the step identification information or the usage device before the one step included in the manufacturing process is started.

### [Supplementary Note 3]

The quality monitoring apparatus according to supplementary note 1 or 2, in which the model switching processing includes processing of switching the state prediction models based on quality information that is one of the step quality information and that is acquired during the step.

### [Supplementary Note 4]

The quality monitoring apparatus according to supplementary note 3, in which, in a case in which the manufacturing process includes a purification process of a target component contained in the liquid, the state of the liquid is a concentration of the target component contained in the liquid, and the quality information acquired during the step includes information that is related to the concentration of the target component in the liquid and that is acquired inline in the step.

### [Supplementary Note 5]

The quality monitoring apparatus according to supplementary note 4, in which the target component is an antibody.

### [Supplementary Note 6]

The quality monitoring apparatus according to any one of supplementary notes 1 to 5, in which the spectroscopic spectrum includes any one of an infrared spectrum, a fluorescence spectrum, or a Raman spectrum.

### [Supplementary Note 7]

The quality monitoring apparatus according to any one of supplementary notes 1 to 6, in which the processor is configured to output a prediction value of the state of the liquid at a first time interval set in advance, based on the spectroscopic spectrum.

### [Supplementary Note 8]

The quality monitoring apparatus according to supplementary note 7, in which the first time interval is 5 seconds or less.

### [Supplementary Note 9]

The quality monitoring apparatus according to supplementary note 8, in which the first time interval is 1 second or less.

### [Supplementary Note 10]

The quality monitoring apparatus according to any one of supplementary notes 7 to 9, in which the processor is configured to acquire the spectroscopic spectrum at a second time interval equal to or shorter than the first time interval, and output the prediction value based on a moving average value of a plurality of the acquired spectroscopic spectra.

### [Supplementary Note 11]

The quality monitoring apparatus according to any one of supplementary notes 7 to 10, in which the processor is configured to output a final quality of the step, indicating a final state of an entire liquid produced in the step, based on the prediction value.

### [Supplementary Note 12]

The quality monitoring apparatus according to supplementary note 11, in which the processor is configured to execute condition determination processing of determining process conditions of a next step based on the final quality of the step.

### [Supplementary Note 13]

The quality monitoring apparatus according to any one of supplementary notes 1 to 12, in which, in a case in which the manufacturing process includes a purification process of a target component contained in the liquid, the state of the liquid is a concentration of the target component contained in the liquid.

### [Supplementary Note 14]

The quality monitoring apparatus according to supplementary note 13, in which the target component is an antibody.

### [Supplementary Note 15]

The quality monitoring apparatus according to any one of supplementary notes 1 to 14, in which the state of the liquid is a state of a protein contained in the liquid.

### [Supplementary Note 16]

An operation method of a quality monitoring apparatus, the operation method comprising: executing monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and model switching processing of switching the state prediction models during the monitoring processing based on step management information for managing at least one step included in the manufacturing process, in which the plurality of state prediction models have been trained before the monitoring processing is started, and the step management information includes at least one of step identification information for identifying at least one step included in the manufacturing process, usage device information of a usage device used in the step, or step quality information that is related to the state of the liquid for each step and that is acquirable in a case in which the step is performed at least once.

### [Supplementary Note 17]

An operation program of a quality monitoring apparatus that causes a computer to function as the quality monitoring apparatus, the operation program causing the computer to execute: monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and model switching processing of switching the state prediction models during the monitoring processing based on step management information for managing at least one step included in the manufacturing process, in which the plurality of state prediction models have been created before the monitoring processing is started, and the step management information includes at least one of step identification information for identifying at least one step included in the manufacturing process, usage device information of a usage device used in the step, or step quality information that is related to the state of the liquid for each step and that is acquirable in a case in which the step is performed at least once.

The technology of the present disclosure can also be combined with various embodiments and/or various modification examples described above, as appropriate. In addition, it goes without saying that the present disclosure is not limited to each of the embodiments described above, various configurations can be adopted as long as the configuration does not deviate from the gist. Further, the technology of the present disclosure includes a storage medium that stores the program in a non-transitory manner, in addition to the program.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Therefore, it goes without saying that unnecessary parts may be deleted, new elements may be added, or replacements may be made with respect to the above-described contents and the above-shown contents within a range that does not deviate from the gist of the technology of the present disclosure. In order to avoid complications and facilitate grasping the parts according to the technology of the present disclosure, in the above-described contents and the above-shown contents, the description of technical general knowledge and the like that do not particularly require description for enabling the implementation of the technology of the present disclosure are omitted.

In the present specification, "A and/or B" has the same meaning as "at least one of A or B". Stated another way, "A and/or B" means that it may be only A, only B, or a combination of A and B. Further, in the present specification, also in a case in which three or more matters are expressed in association by "and/or", the same concept as "A and/or B" is applied.

The disclosure of Japanese Patent Application No. 2023-059234, filed on March 31, 2023, is incorporated in the present specification by reference in its entirety. Furthermore, all of the documents, the patent applications, and the technical standards described in the present specification are incorporated herein by reference to the same extent as in a case in which each document, each patent application, and each technical standard are specifically and individually described by being incorporated in the present specification by reference.

## Claims

1. A quality monitoring apparatus comprising:
a processor configured to execute
monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and
model switching processing of switching the state prediction models during the monitoring processing based on step management information for managing at least one step included in the manufacturing process,
wherein the plurality of state prediction models have been trained before the monitoring processing is started, and the step management information includes at least one of step identification information for identifying at least one step included in the manufacturing process, usage device information of a usage device used in the step, or step quality information that is related to a quality of each step and that is acquirable in a case in which the step is performed at least once.

2. The quality monitoring apparatus according to claim 1,
wherein the model switching processing includes processing of switching the state prediction models based on the step identification information or the usage device before the one step included in the manufacturing process is started.

3. The quality monitoring apparatus according to claim 1,
wherein the model switching processing includes processing of switching the state prediction models based on quality information that is one of the step quality information and that is acquired during the step.

4. The quality monitoring apparatus according to claim 3,
wherein, in a case in which the manufacturing process includes a purification process of a target component contained in the liquid,
the state of the liquid is a concentration of the target component contained in the liquid, and
the quality information acquired during the step includes information that is related to the concentration of the target component in the liquid and that is acquired inline in the step.

5. The quality monitoring apparatus according to claim 4,
wherein the target component is an antibody.

6. The quality monitoring apparatus according to claim 1,
wherein the spectroscopic spectrum includes any one of an infrared spectrum, a fluorescence spectrum, or a Raman spectrum.

7. The quality monitoring apparatus according to claim 1,
wherein the processor is configured to output a prediction value of the state of the liquid at a first time interval set in advance, based on the spectroscopic spectrum.

8. The quality monitoring apparatus according to claim 7,
wherein the first time interval is 5 seconds or less.

9. The quality monitoring apparatus according to claim 8,
wherein the first time interval is 1 second or less.

10. The quality monitoring apparatus according to claim 7,
wherein the processor is configured to acquire the spectroscopic spectrum at a second time interval equal to or shorter than the first time interval, and output the prediction value based on a moving average value of a plurality of the acquired spectroscopic spectra.

11. The quality monitoring apparatus according to claim 7,
wherein the processor is configured to output a final quality of the step, indicating a final state of an entire liquid produced in the step, based on the prediction value.

12. The quality monitoring apparatus according to claim 11,
wherein the processor is configured to execute condition determination processing of determining process conditions of a next step based on the final quality of the step.

13. The quality monitoring apparatus according to claim 1,
wherein, in a case in which the manufacturing process includes a purification process of a target component contained in the liquid,
the state of the liquid is a concentration of the target component contained in the liquid.

14. The quality monitoring apparatus according to claim 13,
wherein the target component is an antibody.

15. The quality monitoring apparatus according to claim 1,
wherein the state of the liquid is a state of a protein contained in the liquid.

16. An operation method of a quality monitoring apparatus, the operation method comprising:
executing
monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and
model switching processing of switching the state prediction models during the monitoring processing based on step management information for managing at least one step included in the manufacturing process,
wherein the plurality of state prediction models have been trained before the monitoring processing is started, and the step management information includes at least one of step identification information for identifying at least one step included in the manufacturing process, usage device information of a usage device used in the step, or step quality information that is related to the state of the liquid for each step and that is acquirable in a case in which the step is performed at least once.

17. An operation program of a quality monitoring apparatus that causes a computer to function as the quality monitoring apparatus, the operation program causing the computer to execute:
monitoring processing of monitoring a quality of a manufacturing process of a biopharmaceutical by using a plurality of state prediction models that predict a state of a liquid produced in the manufacturing process, which is related to the quality of the manufacturing process, using spectroscopic spectrum acquired inline from the liquid as input data, and
model switching processing of switching the state prediction models during the monitoring processing based on step management information for managing at least one step included in the manufacturing process,
wherein the plurality of state prediction models have been created before the monitoring processing is started, and the step management information includes at least one of step identification information for identifying at least one step included in the manufacturing process, usage device information of a usage device used in the step, or step quality information that is related to the state of the liquid for each step and that is acquirable in a case in which the step is performed at least once.
